# EUROPEAN PATENT SPECIFICATION

(11) **EP 2 463 348 B1**
(45) Date of publication and mention of the grant of the patent: **07.10.2015**
(21) Application number: 10806457.7
(22) Date of filing: 03.08.2010
(51) Int. Cl.: C09K 3/18, C09D 5/00, C09D 183/08

(54) **COMPOSITION FOR FORMATION OF WATER-REPELLENT FILM, BASE MATERIAL HAVING WATER-REPELLENT FILM ATTACHED THERETO AND PROCESS FOR PRODUCTION THEREOF, AND ARTICLE FOR TRANSPORT DEVICE**
ZUSAMMENSETZUNG ZUR BILDUNG EINES WASSERABWEISENDEN FILMS, BASISMATERIAL MIT DARAUF ANGEBRACHTEM WASSERABWEISENDEM FILM UND VERFAHREN ZU SEINER HERSTELLUNG SOWIE ARTIKEL FÜR EINE TRANSPORTVORRICHTUNG
COMPOSITION DE FORMATION D'UN FILM HYDROPHOBE, MATÉRIAU DE BASE AYANT UN FILM HYDROPHOBE ATTACHÉ À CELUI-CI ET PROCÉDÉ DE FABRICATION DE CELUI-CI, ET ARTICLE POUR UN DISPOSITIF DE TRANSPORT

(30) Priority: 03.08.2009 JP 2009181047
(43) Date of publication of application: 13.06.2012
(73) Proprietor: Asahi Glass Company, Limited, Tokyo 100-8405 (JP)
(72) Inventor: KISHIKAWA, Noriko, Tokyo 100-8405 (JP); TAKEDA, Yosuke, Tokyo 100-8405 (JP); ITO, Atsushi, Tokyo 100-8405 (JP); YONEDA, Takashige, Tokyo 100-8405 (JP)
(74) Representative: Müller-Boré & Partner Patentanwälte PartG mbB
(86) International application number: PCT/JP2010/063118
(87) International publication number: WO 2011/016458

(56) References cited:
- EP-A1- 1 229 085
- WO-A1-2009/008380
- JP-A- 2002 226 838
- JP-T- 2001 508 120
- JP-T- 2002 506 887
- JP-T- 2005 509 708

## Description

### TECHNICAL FIELD

The present invention relates to a composition for forming a water repellent film which satisfies both a nature (hereinafter referred to as a water droplets-removing property) to minimize deposition of water droplets or to facilitate removal of deposited water droplets, and abrasion resistance and weather resistance; a substrate with a water repellent film having a water repellent layer formed from such a composition for forming a water repellent film and a process for its production; and an article for a transport equipment comprising such a substrate with a water repellent film.

### BACKGROUND ART

Heretofore, in various technical fields, it has been desired to impart a water droplets-removing property to the surface of a substrate. In order to impart a water droplets-removing property to the surface of a substrate, a water repellent treating agent comprising a fluorinated silane compound and a dimethyl silicone compound (Patent Document 1) or a composition for a water repellent film cover comprising a silane compound and an alkyl silane compound (Patent Document 2) has been proposed.

Further, among water repellent articles treated with the above-mentioned water repellent treating agent, an article to be used in the transport field is required to have abrasion resistance and weather resistance in addition to the water droplets-removing property. In order to impart abrasion resistance to a water repellent article, a method of applying a coating solution containing a fluoroalkylsilane and tetraethoxysilane by a flow coating method to form a water repellent coating film having a constant thickness (Patent Document 3) or a water repellent film-covered article containing, in the water repellent film, magnesium oxide, calcium oxide, strontium oxide and boron oxide (Patent Document 4) has, for example, been proposed. Further, in order to impart weather resistance to a water repellent article, a coating composition containing a photostabilizer having hydrolyzable silyl groups (Patent Document 5) has, for example, been proposed. Further, for the purpose of forming a surface-treated layer having a water droplets-removal property as well as abrasion resistance and weather resistance on the surface of a substrate, a water repellent composition containing a silane compound having bonded to silicon a fluorinated organic group having an etheric oxygen bond (Patent Document 6) has been proposed.

However, the techniques disclosed in Patent Documents 1 and 2 have had a problem that the abrasion resistance of the dimethyl silicone compound or the alkyl silane compound is low, and the abrasion resistance of the water repellent article is inadequate. Further, the technique disclosed in Patent Document 3 has had a problem that it is not easy to make the film thickness uniform in a large area, and the technique is not suitable for mass production, and a further problem such that in a case where the surface shape changes during use for a long period of time, the water droplets-removal property tends to deteriorate. Further, the technique disclosed in Patent Document 5 has had a problem that the photostabilizer is practically likely to deteriorate, its effects do not last over a long period of time, and the water droplets-removing property tends to deteriorate.

Accordingly, a water repellent article which is excellent in a water droplets-removal property and further has abrasion resistance and weather resistance, particularly a water repellent article applicable also to a transport equipment, is desired.

Document EP 1 229 085 discloses a water-repellent composition and a substrate coated with said composition. The composition comprises compounds of formula 1 and 4 which are similar to present compounds (A) and (B). In the examples is used a combination of a compound corresponding to present compound (A) wherein R_{f} has 8 carbon atoms, whereas claim 1 is limited to 1-7 carbon atoms, and of a compound corresponding to present compound (B) wherein the perfluoroalkyl chain has 3 carbon atoms, thus within the range of claim 1, and wherein Z is an alkyl chain and W is a fluorinated group which are not according to claim 1.

### PRIOR ART DOCUMENTS

### PATENT DOCUMENTS

Patent Document 1: Japanese Patent No. 3,228,085
Patent Document 2: JP-A-2002-256258
Patent Document 3: Japanese Patent No. 3,342,170
Patent Document 4: WO2001-009266
Patent Document 5: JP-A-2003-138210
Patent Document 6: JP-A-2002-226838

### DISCLOSURE OF INVENTION

### TECHNICAL PROBLEM

It is an object of the present invention to provide a composition for forming a water repellent film, which has an excellent water droplets-removing property and which further has abrasion resistance and weather resistance. Further, another object is to provide a substrate with a water repellent film having a water repellent layer which is formed from such a composition for forming a water repellent film and which is excellent in the water droplets-removing property, abrasion resistance and weather resistance and a process for its production, and an article for a transport equipment.

### SOLUTION TO PROBLEM

The present invention has been made to solve the above problem and provides the following.

The composition for forming a water repellent film of the present invention comprises the following compounds (A) and (B), or a partially hydrolyzed co-condensate of a compound represented by the following formula (1a) and/or its partially hydrolyzed condensate, and a compound represented by the following formula (2a) and/or its partially hydrolyzed condensate,

Compound (A): at least one fluorinated organic silicon compound which contains no etheric oxygen atom and which is selected from the group consisting of a compound represented by the following formula (1a), its partially hydrolyzed condensate, and a compound represented by the following formula (1 b),

R^{f1}-Y-Si(R¹¹)ᵣ(X¹)₃₋ᵣ (1a)

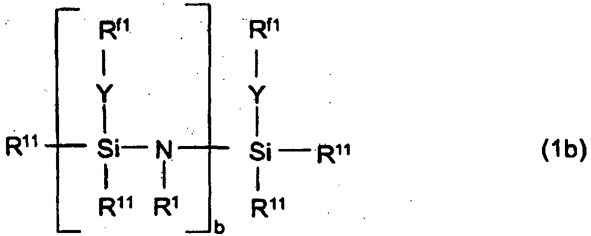

(in the formulae (1a) and (1b), R^{f1} is a C₁₋₇ perfluoroalkyl group having the formula CF₃(CF₂)ₗ-, wherein I is an integer of from 0 to 6, which contains no etheric oxygen atom between carbon-carbon atoms, Y is a C₁₋₆ bivalent organic group which contains no fluorine atom, R¹¹ each independently is a hydrogen atom or a C₁₋₆ hydrocarbon group which contains no fluorine atom, X¹ each independently is a halogen atom, an alkoxy group or an isocyanate group, r is an integer of from 0 to 2, R¹ is a hydrogen atom or a C₁₋₃ hydrocarbon group which contains no fluorine atom, and b is an integer of from 1 to 100)

Compound (B): at least one fluorinated organic silicon compound which contains an etheric oxygen atom and which is selected from the group consisting of a compound represented by the following formula (2a), its partially hydrolyzed condensate, and a compound represented by the following formula (2b),

R^{f2}-W-Z-Si(R¹²)ₚ(X²)₃₋ₚ (2a)

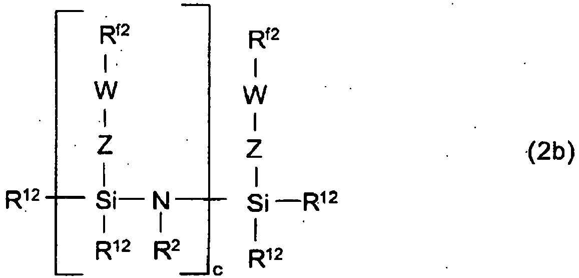

(in the formulae (2a) and (2b), R^{f2} is a C₁₋₇ perfluoroalkyl group having the formula CF₃(CF₂)ₗ-, wherein I is an integer of from 0 to 6, which may have an etheric oxygen atom inserted between carbon-carbon atoms, W is -O-(CF₂CF₂O)ₐ-CF₂- (wherein a is an integer of from 1 to 200), Z is a bivalent organic group selected from -CONHC₃H₆- and -CH₂OCO-NHC3H₆-, R¹² each independently is a hydrogen atom or a C₁₋₆ hydrocarbon group which contains no fluorine atom, X² each independently is a halogen atom, an alkoxy group or an isocyanate group, p is an integer of from 0 to 2, R² is a hydrogen atom or a C₁₋₃ hydrocarbon group which contains no fluorine atom, and c is an integer of from 1 to 100).

Further, the substrate with a water repellent film of the present invention is a substrate with a water repellent film comprising a substrate and a water repellent film formed on at least a part of the surface of the substrate, wherein the water repellent film is composed of at least one layer and has, as the outermost layer, a water repellent layer formed by using the above composition for forming a water repellent film of the present invention.

Further, the present invention provides a process for producing the above substrate with a water repellent film of the present invention, which comprises a step of applying the above composition for forming a water repellent film of the present invention to the surface of a substrate or to the surface of a layer to constitute a lower layer of the outermost layer in the water repellent film, preliminarily formed on the surface of a substrate, followed by curing to form a water repellent layer.

Further, the present invention provides an article for a transport equipment, which comprises the substrate with a water repellent film of the present invention.

### ADVANTAGEOUS EFFECTS OF INVENTION

By using the composition for forming a water repellent film of the present invention, it is possible to form a water repellent film which has an excellent water droplets-removing property and which further has abrasion resistance and weather resistance. Further, the substrate with a water repellent film of the present invention which has a water repellent layer formed by using such a composition for forming a water repellent film, and the article for a transport equipment comprising such a substrate with a water repellent film, are excellent in the water droplets-removing property, abrasion resistance and weather resistance. Further, by the process of the present invention, it is possible to obtain a substrate with a water repellent film which is excellent in the water droplets-removing property, abrasion resistance and weather resistance.

### DESCRIPTION OF EMBODIMENTS

Now, embodiments of the present invention will be described.

### <Composition for forming water repellent film>

The composition for forming a water repellent film of the present invention comprises a compound (A) being at least one fluorinated organic silicon compound which contains no etheric oxygen atom and which is selected from the group consisting of a compound represented by the above formula (1a), its partially hydrolyzed condensate, and a compound represented by the above formula (1b), and a compound (B) being at least one fluorinated organic silicon compound which contains an etheric oxygen atom and which is selected from the group consisting of a compound represented by the above formula (2a), its partially hydrolyzed condensate, and a compound represented by the above formula (2b). Or, the composition for forming a water repellent film of the present invention comprises a partially hydrolyzed co-condensate of a compound represented by the above formula (1a) and/or its partially hydrolyzed condensate, and a compound represented by the above formula (2a) and/or its partially hydrolyzed condensate.

The excellent water droplets-removing property of the water repellent film (the water repellent layer in the substrate with a water repellent film of the present invention) formed by using the composition for forming a water repellent film of the present invention, is attributable to the molecular structure of the above compound (B). More specifically, the fluorinated organic group containing an etheric oxygen atom of the compound (B) contributes to the improvement of the mobility or falling off property of water droplets on the coating film surface in the above water repellent film. Further, by incorporating the above compound (A) to the composition for forming a water repellent film of the present invention, the water droplets-removing property of the water repellent film and its abrasion resistance and weather resistance are improved.

Here, with respect to the above effect of the fluorinated organic group containing an etheric oxygen atom, of the compound (B), a detailed mechanism is not clearly understood, but it is considered that fluorine atoms in the fluorinated organic group containing an etheric oxygen atom serve to reduce the surface energy of the above water repellent film, and a high molecular chain mobility derived from the structure linked by the etheric oxygen atom contributes to an effective alignment of fluorine atoms in the water repellent film. That is, it is considered that the high molecular chain mobility makes a change in alignment of fluorine atoms possible to facilitate movement of water droplets on the surface of the water repellent film, and consequently, the falling off property of water droplets is increased.

Further, also the fluorinated organic group having no etheric oxygen atom in a surface treating agent which has been heretofore proposed, contains fluorine atoms and thus is able to reduce the surface energy of the treated layer formed from such a surface treating agent. However, a fluorinated organic group having no etheric oxygen atom has a very rigid structure, whereby fluorine atoms present on the surface tend to hardly undergo an alignment change and thus cannot follow the movement of the water droplets. Therefore, it is considered that water droplets may well fall off in a certain region, but tend to hardly fall off in another region, so that water droplets are hooked somewhere, thus leading to deterioration of the falling off property of water droplets.

Now, the compound (A) and the compound (B) contained in the composition for forming a water repellent film of the present invention will be described.

The compound (A) which is contained in the composition for forming a water repellent film of the present invention, is at least one fluorinated organic silicon compound which contains no etheric oxygen atom and which is selected from the group consisting of a compound represented by the following formula (1a), its partially hydrolyzed condensate, and a compound represented by the following formula (1b).

A partially hydrolyzed condensate of the compound represented by the following formula (1a) will be described later. Firstly, the compound (A) which is not a partially hydrolyzed condensate, will be described.

R^{f1}-Y-Si(R¹¹)ᵣ(X¹)₃₋ᵣ (1a)

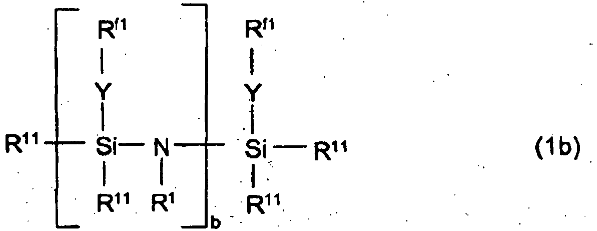

The compound (A) may be composed solely of the compound represented by the above formula (1a), may be composed solely of the compound represented by the above formula (1b), or may be composed of their mixture.

In each of the above formulae (1a) and (1b), R^{f1} is the following C₁₋₇ perfluoroalkyl group which contains no etheric oxygen atom between carbon-carbon atoms:

CF₃(CF₂)ₗ-,

Here, I is an integer of from 0 to 6.

In each of the formulae (1a) and (1b), Y being a group which links R^{f1} and a silicon atom, is a C₁₋₆ bivalent organic group which contains no fluorine atom and is not particularly limited other than such a condition. Y is preferably a bivalent organic group selected from -(CH₂)ᵢ- (wherein i is an integer of from 1 to 6),-CONH(CH₂)ⱼ-(wherein j is an integer of from 1 to 5) and -CONH(CH₂)_{q}NH(CH₂)₅₋ₖ- (wherein k is an integer of from 1 to 4), more preferably -(CH₂)₂-, -CONH(CH₂)₃-, -CONH(CH₂)₂NH(CH₂)₃-.

In each of the formulae (1a) and (1b), R¹¹ each independently is a hydrogen atom or a C₁₋₆ hydrocarbon group which contains no fluorine atom. Among them, in the formula (1a), R¹¹ is preferably a C₁₋₄ hydrocarbon group, particularly preferably a methyl group or an ethyl group. Further, in the formula (1b), R¹¹ is preferably a hydrogen atom.

In the formula (1a), X¹ is a halogen atom, an alkoxy group or an isocyanate group. Each of them is a hydrolyzable group. When r is 0 or 1, a plurality of X¹ may be the same or different. Further, X¹ is preferably a chlorine atom, a C₁₋₄ alkoxy group or an isocyanate group, particularly preferably a chlorine atom. r is an integer of from 0 to 2, preferably 0 or 1, since the adhesion, and the durability of the formed layer will be thereby excellent.

In the formula (1b), R¹ each independently is a hydrogen atom or a C₁₋₃ hydrocarbon group which contains no fluorine atom. R¹ is preferably a hydrogen atom with a view to improvement of the reactivity.

In the formula (1b), b is an integer of from 1 to 100. Here, b represents the number of units of the silicon-nitrogen bond in the compound represented by the formula (1b), and in the present invention, b is preferably from 1 to 50 from the viewpoint of the coating property.

In the composition for forming a water repellent film of the present invention, one type of the compound (A) may be used alone, or two or more types of the compound (A) may be used in combination.

A specific example of the compound represented by the formula (1b) is:

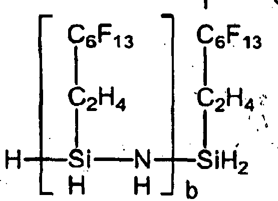

The above compound (A) to be used in the present invention can be produced by a common method. Further, as the compound (A), a commercial product is available, and therefore, in the present invention, it is possible to employ such a commercial product.

The compound (B) contained in the composition of the present invention is at least one fluorinated organic silicon compound which contains an etheric oxygen atom and which is selected from the group consisting of a compound represented by the following formula (2a), its partially hydrolyzed condensate, and a compound represented by the following formula (2b). The partially hydrolyzed condensate of the compound represented by the following formula (2a) will be described later. Firstly, the compound (B) which is not a partially hydrolyzed condensate, will be described.

R^{f2}-W-Z-Si(R¹²)ₚ(X²)₃₋ₚ (2a)

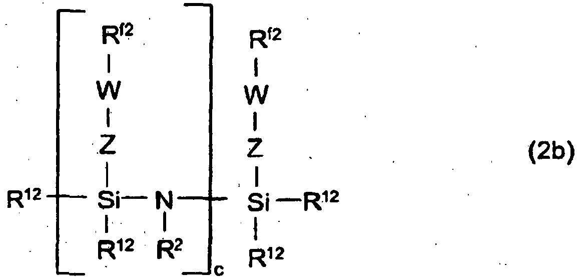

The compound (B) may be composed solely of the compound represented by the above formula (2a), may be composed solely of the compound represented by the above formula (2b), or may be composed of their mixture.

In each of the above formulae (2a) and (2b), R^{f2} is a C₁₋₇ perfluoroalkyl group, which may have an etheric oxygen atom inserted between carbon-carbon atoms). As such R^{f2}, the following groups may specifically be mentioned.

CF₃(CF₂)ₗ-,

Here, I is an integer of from 0 to 6.

The perfluoroalkyl group having an etheric oxygen atom inserted between carbon-carbon atoms is a group having an etheric oxygen atom inserted between carbon-carbon atoms of the above-mentioned perfluoroalkyl group. Depending upon the inserted etheric oxygen atom, in a case where a perfluoro(oxyethylene) group i.e. -OCF₂CF₂- is formed on the bond terminal side in the perfluoroalkyl group, such a perfluoro(oxyethylene) group is regarded as a perfluoro(oxyethylene) group in W in the above formula. In a case where such a perfluoro(oxyethylene) group is not linked to the perfluoro(oxyethylene) group in W, it is a perfluoro(oxyethylene) group in R¹². The etheric oxygen atom in R¹² may form a perfluoro(oxypropylene) group, but a perfluoro(oxypropylene) group may not be able to sufficiently exhibit the desired effect due to the presence of a trifluoromethyl group as its side chain. Therefore, in a case where there are two or more etheric oxygen atoms inserted in R^{f2}, they preferably form a structure wherein two or more units of perfluoro(oxyethylene) groups are repeated.

It is preferred that a perfluoroalkyl group having an etheric oxygen atom inserted does not have a -OCF₂O- structure. This means that the -OCF₂O- structure is a structure, on which the presence of a nuclear structure cannot be detected by a usual analytical method (such as ¹⁹F-NMR (nuclear magnetic resonance)). In a case where R^{f2} has a structure wherein two or more units of perfluoro(oxyethylene) groups are repeated, -OCF₂O- is likely to be formed at one end of such a structure in many cases. However, the -OCF₂O- structure in R^{f2} is unstable and is likely to bring about deterioration of the heat resistance.

When etheric oxygen atoms are inserted between carbon-carbon atoms, the number of oxygen atoms to be inserted is preferably from 1 to 7, more preferably from 1 to 4. The positions of such oxygen atoms to be inserted are between carbon atom-carbon atom single bonds, and the number of carbon atoms present between oxygen atoms is at least 2.

In each of the above formulae (2a) and (2b), W is a bivalent organic group represented by -O-(CF₂CF₂O)ₐ-CF₂-, wherein a is an integer of from 1 to 200. Here, a is preferably an integer of from 3 to 50, more preferably from 4 to 25, further preferably from 5 to 10. By adjusting the number for a within this range, it becomes possible to impart a sufficient water droplets-removing property to the water repellent film which is formed from the composition for forming a water repellent film of the present invention.

In each of the above formulae (2a) and (2b), Z is a bivalent organic group selected from -CONHC₃H₆- and -CH₂OCONHC₃H₆-.

In each of the above formulae (2a) and (2b), R¹² may be the same groups as R¹¹ in the above-mentioned formulae (1a) and (1b). Its preferred examples are also the same as mentioned above.

In the formula (2a), X² may be the same group as X¹ in the above formula (1a). Its preferred examples are also the same as mentioned above.

In the formula (2a), p is an integer of from 0 to 2, but is preferably 0 or 1, since the adhesion and the durability of the formed layer will thereby be excellent.

In the formula (2b), R² may be the same group as R¹ in the above formula (1b). Its preferred examples are also the same as mentioned above. c is one representing the number of units of silicon-nitrogen bonds in the compound represented by the formula (2b), and in the present invention, c is preferably from 1 to 50 from the viewpoint of the coating property.

In the composition for forming a water repellent film of the present invention, one type of the compound (B) may be used alone, or two or more types may be used in combination.

Specific examples of the compound (B) will be shown below with respect to each of the compound represented by the formula (2a) and the compound represented by the formula (2b).
(Compounds represented by the formula (2a))

R^{f2}-O-(CF₂CF₂O)ₐ-CF₂-CONHC₃H₆Si(R¹²)ₚ(X²)₃₋ₚ (B1)

R^{f2}-O-(CF₂CF₂O)ₐ-CF₂-CH₂OCONHC₃H₆Si(R¹²)p(X²)₃₋ₚ (B2)

R^{f2}-O-(CF₂CF₂O)ₐ-CF₂-CH₂OC₃H₆Si(R¹²)ₚ(X²)₃₋ₚ (B3)

R^{f2}-O-(CF₂CF₂O)ₐ-CF₂-CF₂OC₃H₆Si(R¹²)ₚ(X²)₃₋ₚ (B4)

R^{f2}-O-(CF₂CF₂O)ₐ-CF₂-C₂H₄Si(R¹²)ₚ(X²)₃₋ₚ (B5)

R^{f2}-O-(CF₂CF₂O)ₐ-CF₂-C₃H₆Si(R¹²)ₚ(X²)₃₋ₚ (B6)

(Compounds represented by the formula (2b))

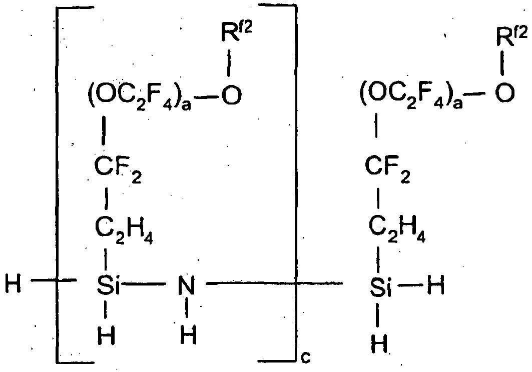

Among them, preferred specific examples as the compound (B) in the present invention, are as follows.

CF₃-O-(CF₂CF₂O)ₐ-CF₂-CONHC₃H₆Si(OCH₃)₃

CF₃-O-(CF₂CF₂O)ₐ-CF₂-CONHC₃H₆Si(OC₂H₅)₃

CF₃-O-(CF₂CF₂O)ₐ-CF₂-CONHC₂H₄Si(OCH₃)₃

CF₃-O-(CF₂CF₂O)ₐ-CF₂-CONHC₂H₄Si(OC₂H₅)₃

CF₃-O-(CF₂CF₂O)ₐ-CF₂-C₂H₄Si(OCH₃)₃

CF₃-O-(CF₂CF₂O)ₐ-CF₂-C₂H₄Si(OC₂H₅)₃

(In all of the above compounds (B), a=7 to 8, and the average value of a is 7.3 (hereinafter referred to as "a=7 to 8, average value: 7.3".))

The above compound (B) to be used in the present invention can be produced by a known method. For example, the above compounds (B1) to (B6) can be produced specifically by the method disclosed in WO2009-008380.

The compound represented by the formula (1a) and the compound represented by the formula (2a) may be their respective partially hydrolyzed condensates. A partially hydrolyzed condensate is meant for an oligomer to be formed by hydrolysis of all or some of hydrolyzable silyl groups in a solvent in the presence of a catalyst such as an acid catalyst or an alkali catalyst, followed by dehydration condensation. However, the condensation degree (oligomerization degree) of such a partially hydrolyzed condensate is required to be such a degree that the product is soluble in a solvent. Therefore, the compound (A) to be contained in the composition for forming a water repellent film of the present invention may be a partially hydrolyzed condensate of the compound represented by the formula (1a), and likewise, the compound (B) may be a partially hydrolyzed condensate of the compound represented by the formula (2a). Further, they may contain the respective unreacted compounds represented by the formulae (1a) and (2a).

The composition for forming a water repellent film of the present invention comprises the above compounds (A) and (B).

Here, the compounds (A) and (B) may be contained in the form of the above-described compounds themselves in the composition for forming a water repellent film of the present invention. The composition for forming a water repellent film of the present invention preferably contains the compound (A) in the form of the compound represented by the formula (1a) and/or its partially hydrolyzed condensate, and the compound (B) in the form of the compound represented by the formula (2a) and/or its partially hydrolyzed condensate. In such a case, it is more preferred that they are contained in the form of a partially hydrolyzed co-condensate of the compounds (A) and (B).

As mentioned above, the partially hydrolyzed co-condensate of the compounds (A) and (B) is also meant for an oligomer to be formed by hydrolysis of all or some of hydrolyzable silyl groups in a solvent in the presence of a catalyst such as an acid catalyst or an alkali catalyst, followed by dehydration condensation, but here, the oligomer is one obtainable by hydrolytic condensation of a mixture of two types of hydrolyzable silyl group-containing compounds (i.e. the compound (A): a compound represented by the formula (1a) and/or its partially hydrolyzed condensate, and the compound (B): a compound represented by the formula (2a) and/or its partially hydrolyzed condensate), and therefore is called as a partially hydrolyzed "co-" condensate. The condensation degree (oligomerization degree) of such a partially hydrolyzed co-condensate is required to be such that the product is soluble in a solvent.

Further, the partially hydrolyzed co-condensate is one to be formed by reacting, as the compound (A), a compound represented by the formula (1a) and/or its partially hydrolyzed condensate, and, as the compound (B), a compound represented by the formula (2a) and/or its partially hydrolyzed condensate in a solvent containing them, and may contain unreacted compounds (A) and (B). In the case of producing the partially hydrolyzed co-condensate, it is preferred that as the compound (A), a compound represented by the formula (1a) (not its partially hydrolyzed condensate) is used, and as the compound (B), a compound represented by the formula (2a) (not its partially hydrolyzed condensate) is used.

The partially hydrolyzed co-condensate of the compounds (A) and (B) can be produced by dissolving in a solvent a prescribed amount of a compound represented by the formula (1a) and/or its partially hydrolyzed condensate, and a prescribed amount of a compound represented by the formula (2a) and/or its partially hydrolyzed condensate, followed by stirring for a prescribed time in the presence of a catalyst such as an acid catalyst or an alkali catalyst, and water. As the acid catalyst, hydrochloric acid, nitric acid, acetic acid, sulfuric acid, phosphoric acid, sulfonic acid, methane sulfonic acid or p-toluene sulfonic acid may, for example, be used. As the alkali catalyst, sodium hydroxide, potassium hydroxide or aqueous ammonia may, for example, be used. By using an aqueous solution of such a catalyst, water required for the hydrolysis may be present in the reaction system. By heating in the presence of the catalyst and water, the reaction may be accelerated, but if the reaction proceeds too much, the condensation degree becomes too high, and the product is likely to be insoluble in a solvent. So long as a proper amount of the catalyst is present, it is preferred to carry out the reaction at normal temperature. The obtained solution of the partially hydrolyzed co-condensate may be used as it is, as the composition for forming a water repellent film of the present invention.

By using the above partially hydrolyzed co-condensate, it is possible to form a water repellent film having a higher performance. For example, in the case of a water repellent film which is formed from the compound represented by the formula (1a) and the compound represented by the formula (2a), the water repellent film is made of a hydrolyzed co-condensate of the two compounds and becomes a film wherein units derived from the two compounds are uniformly dispersed. The hydrolyzed co-condensate of the two compounds can be formed in a relatively short time, and in a film directly formed from the compound represented by the formula (1a) and the compound represented by the formula (2a), it is likely that the uniformity in the distribution of the units derived from the two compounds tends to deteriorate. By preliminarily preparing the partially hydrolyzed co-condensate containing units derived from the two compounds, such uniformity is considered to be improved.

The compositional proportions of effective components in the composition for forming a water repellent film of the present invention can be determined from the amounts of the compounds (A) and (B) to be used. In a case where the composition comprises the compounds (A) and (B), the compositional proportions can be determined by the proportions of the two compounds used for producing the composition. However, in a case where the composition for forming a water repellent film of the present invention contains the above-mentioned partially hydrolyzed co-condensate, it is difficult to measure the compositional proportions of the effective components in such a partially hydrolyzed co-condensate. In such a case, in the present invention, the compositional proportions of the effective components are determined by the starting material composition before producing the partially hydrolyzed co-condensate. That is, the compositional proportions of the effective components are determined from the amounts of the compounds (A) and (B) used as starting materials for the partially hydrolyzed co-condensate. For example, in a case where the composition for forming a water repellent film is formed by producing a partially hydrolyzed co-condensate from the compound represented by the formula (1a) and the compound represented by the formula (2a), the compositional proportions of units of the compound represented by the formula (1 a) and units of the compound represented by the formula (2a) in the partially hydrolyzed co-condensate are regarded to be the same as the compositional proportions of the two starting material compounds used.

The proportion of the compound (B) in the composition for forming a water repellent film of the present invention is preferably from 10 to 90 mass%, more preferably from 10 to 60 mass%, particularly preferably from 10 to 30 mass%, as the mass percentage of the compound (B) to the total mass of the compounds (A) and (B) represented by [compound (B)]/[compounds (A) and (B)]×100.

Further, in such a case, the proportion of the compound (A) in the composition for forming a water repellent film of the present invention is preferably from 90 to 10 wt%, more preferably from 90 to 40 mass%, particularly preferably from 90 to 70 mass%, as mass percentage of the compound (A) to the total mass of the compounds (A) and (B). As mentioned above, in the case of the partially hydrolyzed co-condensate, the mass percentage here is a compositional proportion calculated from the amounts of the compounds (A) and (B) before the reaction.

The composition for forming a water repellent film of the present invention may be composed solely of the essential components i.e. the above compounds (A) and (B), or the partially hydrolyzed co-condensate of the compound represented by the formula (1a) and/or its partially hydrolyzed condensate, and the compound represented by the formula (2a) and/or its partially hydrolyzed condensate (which may contain the compound (A) and/or the compound (B), as the case requires), but in consideration of e.g. the economical efficiency, operation efficiency, control ability of the thickness of the obtainable water repellent film, it usually contains an organic solvent. The organic solvent is not particularly limited so long as it dissolves the essential components. The organic solvent is preferably an alcohol, an ether, a ketone, an aromatic hydrocarbon, a paraffin type hydrocarbon, an acetic acid ester or the like, particularly preferably an organic solvent containing fluorine atoms (such as a fluoroalcohol or a fluorohydrocarbon). The organic solvent is not limited to one type only, and two or more types of solvents different in the polarity or evaporation rate, may be used as mixed.

Further, in a case where the composition for forming a water repellent film of the present invention contains partially hydrolyzed condensates or partially hydrolyzed co-condensates, it may contain a solvent used for producing such condensates, and such a solvent may be the same as the organic solvent for the composition for forming a water repellent film. Further, the composition for forming a water repellent film may contain a component such as a catalyst used for the partially hydrolytic condensation. Particularly, the composition for forming a water repellent film containing the partially hydrolyzed co-condensate is preferably the solution of the partially hydrolyzed co-condensate obtained by the production of the partially hydrolyzed co-condensate, itself.

The proportion of the organic solvent in the composition for forming a water repellent film of the present invention is preferably at most 100,000 parts by mass, particularly preferably at most 10,000 parts by mass, per 100 parts by mass of the total mass of the compounds (A) and (B). If the proportion exceeds 100,000 parts by mass, treatment irregularities may sometimes be formed in the obtainable water repellent film.

The composition for forming a water repellent film of the present invention may contain a functional additive as an optional component depending upon the particular purpose within a range not to impair the effects of the present invention. Such a functional additive is preferably selected in consideration of the reactivity or compatibility with the essential components, etc., and a non-fluorinated water repellent material such as one terminal-reactive polydimethyl siloxane or both terminal reactive polydimethyl siloxane, ultrafine particles of metal oxide such as silica, alumina, zirconia or titania, a coloring material such as a dye or pigment, an anti-fouling material, a curing catalyst or various resins may, for example, preferably be mentioned. The amount of such a functional additive to be added is preferably from 0.01 to 20 parts by mass, per 100 parts by mass of the solid content (the components excluding volatile components such as organic solvents) of the composition for forming a water repellent film. An excessive addition of the functional additive to the composition for forming a water repellent film is likely to bring about deterioration of the performance such as the water droplets-removing property of the obtainable water repellent film.

Further, even in a case where a partially hydrolyzed condensate or a partially hydrolyzed co-condensate is not contained, in the composition for forming a water repellent film of the present invention, it is also preferred to incorporate a catalyst such as an acid catalyst in order to promote a hydrolytic co-condensation reaction of the compounds (A) and (B). Even in a case where a partially hydrolyzed condensate or a partially hydrolyzed co-condensate is contained, in a case where the catalyst used for the preparation of such a condensate is not remained in the composition for forming a water repellent film, it is preferred to incorporate the catalyst. As such a catalyst, an acid catalyst is preferred. By the presence of the catalyst, it is possible to form a water repellent film having good abrasion resistance and weather resistance.

As a method for forming a water repellent film by using the composition for forming a water repellent film of the present invention, it is possible to employ a known method in the surface treatment with a fluorinated organosilane compound type surface treating agent. For example, the above composition is applied to the surface of a substrate by a method such as brush coating, flow coating, spin coating, dip coating, squeegee coating, spray coating or manual coating, dried as the case requires in the atmospheric air or in a nitrogen atmosphere and then cured to form a water repellent film. The conditions for curing may be suitably set depending upon the type, concentration, etc., of the composition for forming a water repellent film to be used, but preferred conditions may, for example, be conditions of a temperature of from 20 to 50°C and a humidity of from 50 to 90% RH. The time for curing varies depending upon the type, concentration, curing conditions, etc. of the composition for forming a water repellent film to be used, but is usually preferably from 1 to 72 hours. Depending upon the treating method, an excess component may be generated to impair the appearance quality, but in such a case, the appearance may be adjusted by removing such an excess component by e.g. wiping with or without a solvent. The thickness of the water repellent film to be formed from the composition for forming a water repellent film of the present invention is not particularly limited, but in consideration of the economical efficiency, the thickness is preferably at most 50 nm, and the lower limit is the thickness of a monomolecular layer.

### <Substrate with a water repellent film and process for its production>

The substrate with a water repellent film of the present invention is a substrate with a water repellent film comprising a substrate and a water repellent film formed on at least a part of the surface of the substrate, wherein the water repellent film is composed of at least one layer and has, as the outermost layer, a water repellent layer formed by using the composition for forming a water repellent film of the present invention.

The substrate to be used for the substrate with a water repellent film of the present invention is not particularly limited so long as it is a substrate made of a material which is usually required to impart water repellency, and a substrate made of a metal, plastic, glass, ceramics or a combination thereof (such as a composite material, of a laminated material) is preferably used. Particularly preferred is a transparent substrate made of e.g. glass or plastic. The glass may, for example, be usual soda lime glass, borosilicate glass, alkali-free glass or quartz glass. Among them, soda lime glass is particularly preferred. Further, the plastic may, for example, be an acrylic resin such as polymethyl methacrylate, an aromatic polycarbonate resin such as polyphenylene carbonate, or an aromatic polyester resin such as polyethylene terephthalate (PET), and among them, polyethylene terephthalate (PET) is preferred.

The shape of the substrate may be a flat plate, or its entire surface or a part thereof may have a curvature. The thickness of the substrate may suitably be selected depending upon the particular application of the substrate with a water repellent films, but it is usually preferably from 1 to 10 mm.

As the substrate to be used for the present invention, depending upon the particular purpose, one having its surface treated by acid treatment (treatment with e.g. diluted hydrofluoric acid, sulfuric acid, hydrochloric acid), alkali treatment (treatment with an aqueous sodium hydroxide solution) or discharge treatment (such as plasma irradiation, corona irradiation, or electron beam irradiation) may be used. Further, the substrate may be one having, on its surface, a vapor deposition film, a sputtered film or various films formed by e.g. a wet method. In a case where the substrate is soda lime glass, it is preferred from the viewpoint of durability to provide a film to prevent elution of Na ions. In a case where the substrate is glass produced by a float process, it is preferred from the viewpoint of durability to form a water repellent film on the top surface where the amount of tin on the surface is small.

In the substrate with a water repellent film of the present invention, the water repellent film to be formed on at least a part of the surface of the substrate, is composed of at least one layer and has, as the outermost layer, a layer made of a water repellent film (water repellent layer) formed by using the composition for forming a water repellent film of the present invention.

Such a water repellent film in the present invention may be composed solely of the water repellent layer, or may have, in addition to the water repellent layer, a layer other than the water repellent layer depending upon the particular purpose. In any case, the water repellent layer of the substrate with a water repellent film of the present invention is one to be formed as the outermost layer of the water repellent film. The water repellent layer as the outermost layer of the water repellent film may be formed on the substrate as the outermost layer of the water repellent film by the above described method by using the composition for forming a water repellent film of the present invention. That is, as a process for producing the substrate with a water repellent film in a case where in the substrate with a water repellent film of the present invention, the water repellent film is composed solely of the water repellent layer, a process having a step of forming a water repellent layer by applying the composition for forming a water repellent film of the present invention on the surface of a substrate, followed by curing, may be mentioned.

Further, as a process for producing the substrate with a water repellent film in a case where the water repellent film has a layer other than the water repellent layer, a process having a step of forming all layers other than a water repellent layer on the surface of a substrate, and then applying the composition for forming a water repellent layer in the same manner as described above on the surface of a layer being a lower layer of the water repellent layer (the outermost layer), followed by curing to form a water repellent layer, may be mentioned. That is, it is a process having a step of forming a water repellent layer by applying and curing the composition for forming a water repellent film on the surface of a layer being a lower layer of the outermost layer in the water repellent film, preliminarily formed on the substrate surface.

The thickness of the water repellent layer in the water repellent layer-attached substrate of the present invention is not particularly limited so long as it is a thickness to satisfy both a sufficient water droplets-removing property and durability such as abrasion resistance, weather resistance, etc. Such a thickness of the water repellent layer is preferably from 2 to 30 nm, more preferably from 5 to 20 nm in a case where the above water repellent film is composed solely of the water repellent layer. Further, also in a case where the above water repellent film has a layer other than the water repellent layer, e.g. the following interlayer, the thickness of the water repellent layer is preferably from 2 to 30 nm, more preferably from 5 to 20 nm. Here, the thickness of the water repellent layer may suitably be controlled by e.g. the concentration of the composition for forming a water repellent layer to be used, the coating conditions, the heating conditions, etc.

The substrate with a water repellent film of the present invention is preferably such that the above water repellent film further has, as a layer other than the above water repellent layer, an interlayer composed mainly of silica between the substrate and the water repellent layer. By providing such an interlayer, adhesion between the water repellent film and the substrate increases, or the denseness as the entire water repellent film is increased, so that it becomes possible to improve the durability such as the abrasion resistance, weather resistance, etc.

The interlayer composed mainly of silica, which the water repellent film has, can be formed, specifically, by using a composition for forming an interlayer which contains at least one compound (C) selected from the group consisting of a compound represented by the following formula (3), its partially hydrolyzed condensate and a perhydropolysilazane.

Si(X³)₄ (3)

In the above formula (3), X³ is a halogen atom, an alkoxy group or an isocyanate group, and the plurality of X³ may be the same or different. Among them, X³ is preferably a chlorine atom, a C₁₋₄ alkoxy group or an isocyanate group, and four X³ are preferably the same.

As such a compound represented by the formula (3), specifically, Si(NCO)₄, Si(OCH₃)₄ or Si(OC₂H₅)₄ is, for example, preferably used. Further, their partially hydrolyzed condensates may be obtained by a method similar to the one described in the production of partially hydrolyzed condensates of compounds represented by the above formulae (1a) and (2a). Further, commercial products are available as the compound represented by the formula (3) and its partially hydrolyzed condensate, and in the present invention, such commercial products may be employed.

A perhydropolysilazane is a linear or cyclic oligomer having a structure represented by -SiH₂-NH-SiH₂-, and the number of silicon atoms per molecule is preferably from 2 to 500. For example, in the above formula (1b), it is a compound having a structure wherein all of the groups bonded to silicon atoms and nitrogen atoms become hydrogen atoms. A commercial product is available as the perfluoropolysilazane, and in the present invention, such a commercial product may be used.

Further, for the composition for forming the interlayer, one type of the compound (C) may be used alone, or two or more types may be used in combination.

The composition for forming an interlayer to form an interlayer which the water repellent film has in the substrate with a water repellent film of the present invention, further contains, in addition to the compound (C), the compound (A) being at least one fluorinated organic silicon compound which contains no etheric oxygen atom and which is selected from the group consisting of a compound represented by the above formula (1a) and the compound represented by the above formula (1b). As the compound (A) in the composition for forming an interlayer, specifically, the same compound as the compound (A) described in the composition for forming a water repellent film may be mentioned, and also with respect to the preferred compound (A), the same compound as the preferred compound to be used for the composition for forming a water repellent film may be mentioned. In the composition for forming an interlayer, one type of the compound (A) may be used alone, or two or more types may be used in combination.

Further, with respect to the compound (C) and (A), the respective compounds as described above may be contained, as they are, in the composition for forming an interlayer, or they may be contained in the form of a partially hydrolyzed co-condensate of the compound represented by the above formula (3) and/or its partially hydrolyzed condensate, as the compound (C), and the compound represented by the above formula (1a) and/or its partially hydrolyzed condensate, as the compound (A). Such a partially hydrolyzed co-condensate is one to be contained instead of the compound (C) in the composition for forming an interlayer, but the composition for forming an interlayer may contain the compound (C) together with such a partially hydrolyzed co-condensate. Likewise, the compound (A) may be contained together with such a partially hydrolyzed co-condensate. The partially hydrolyzed co-condensate of the above compounds (C) and (A) may be produced, in the same manner as the partially hydrolyzed co-condensate of the compounds (A) and (B), from the compound represented by the above formula (3) and/or its partially hydrolyzed condensate, as the compound (C), and the compound represented by the above formula (1a) and/or its partially hydrolyzed condensate, as the compound (A).

Here, the concept of the water repellent film in the present invention is not such that the boundary line can strictly be drawn, but between adjacent layers, microscopically, part or all of the interface may mutually be mixed. In the water repellent film of the substrate with a water repellent film, by providing an interlayer between the water repellent layer and the substrate, it is possible to improve the durability of the water droplets-removing property. Details of this mechanism are not clearly understood, but it is considered that when the composition for forming an interlayer contains the compound (A), the obtainable interlayer is effective to prevent penetration into the layer of various deteriorating factors accompanying water by its high water repellent effect, and when the compound (C) is contained, by its excellent reactivity, the obtainable interlayer is effective to improve the adhesion between the substrate and the water repellent layer formed from the composition for forming a water repellent layer, and further, by the effect of the reactive group of the compound (C), the denseness of the obtainable interlayer is increased, whereby the denseness as the entire water repellent film is also increased, thus contributing to improvement of the durability.

The proportion of the compound (A) in the composition for forming an interlayer to be used in the present invention, is preferably from 5 to 70 mass%, more preferably from 5 to 50 mass%, as the mass percentage of the compound (A) to the total mass of the compounds (A) and (C) represented by [compound (A)]/[compounds (A) and (C)]×100. In such a case, the proportion of the compound (C) in the composition for forming an interlayer of the present invention, is preferably from 95 to 30 wt%, more preferably from 95 to 50 mass%, as the mass percentage of the compound (C) to the total mass of the compounds (A) and (C). Further, in a case where the composition for forming an interlayer contains the partially hydrolyzed co-condensate, the mass percentage here is, like the proportions of the compounds (A) and (B) in the composition for forming a water repellent film, the mass percentage calculated by using the amounts of the compounds (C) and (A) before the hydrolytic co-condensation reaction, with respect to the partially hydrolyzed co-condensate.

The composition for forming an interlayer may be composed solely of the compound (C) or solely of the compounds (C) and (A), or may be composed solely of the partially hydrolyzed co-condensate of the compound represented by the above formula (3) and/or its partially hydrolyzed condensate, and the compound represented by the formula (1a) and/or its partially hydrolyzed condensate (which may contain the compound (C) and/or the compound (A), as the case requires), but it usually contains an organic solvent in consideration of e.g. economical efficiency, working efficiency, efficiency for control of the thickness of the treated layer.

As the organic solvent, the organic solvent described in the composition for forming a water repellent film may preferably be used. Also the amount of the organic solvent to be used for the composition for forming an interlayer may be similar to the case of the above-described composition for forming a water repellent film. That is, the amount the organic solvent to be used for the composition for forming an interlayer is preferably an amount of at most 100,000 parts by mass, more preferably an amount of 10,000 parts by mass, per 100 parts by mass of the solid content of the composition, e.g. per 100 parts by mass of the mass of the compound (C) in the case where only the compound (C) is contained as the solid content, or per 100 parts by mass of the total mass in a case where only the compounds (C) and (A) are contained as the solid content. If the amount exceeds 100,000 parts by mass, there may be a case where treatment irregularities are observed in the obtainable water repellent film.

The amount of the organic solvent to be used for the composition for forming an interlayer is more preferably at most 3,500 parts by mass, particularly preferably at most 2,000 parts by mass, per 100 parts by mass of the total mass of the solid content contained in the composition. By reducing the amount of the organic solvent to the solid content contained in the composition for forming an interlayer in such a manner, it becomes easy to increase the thickness of the interlayer to be formed by using the composition, and thus to contribute to an improvement of the alkali resistance, salt water resistance, etc. of the water repellent film. Further, the lower limit of the amount of the organic solvent to be used for the composition for forming an interlayer is not particularly limited, but as mentioned above, it is preferred to set the lower limit to be 500 parts by mass per 100 parts by mass of the total mass of the solid content contained in the composition, in consideration of the economical efficiency, working efficiency, efficiency for controlling the thickness of the treated layer.

Further, the composition for forming an interlayer may contain a functional additive depending upon the particular purpose within a range not to impair the effects of the present invention. As such a functional additive, one described in the composition for forming a water repellent film may be preferably mentioned. Further, like the above-mentioned composition for forming a water repellent film, the composition for forming an interlayer may contain a component such as an acid catalyst, as the case requires. Further, also with respect to the process for forming an interlayer by using the composition for forming an interlayer, a process similar to a process for forming a water repellent film described in the composition for forming a water repellent film of the present invention may preferably be mentioned.

The thickness of the interlayer to be formed from the composition for forming an interlayer is not particularly limited, but if it is too thick, a damage tends to be distinct, and therefore, it is preferably at most 50 nm. The lower limit is the thickness of a monomolecular layer. The thickness of the interlayer may suitably be controlled by e.g. the concentration of the composition for forming an interlayer, the coating conditions, the heating conditions. Further, the thickness of the entire water repellent film is preferably from 2 to 100 nm, more preferably from 5 to 20 nm in consideration of the maintenance of the functions of the water repellent film in the present invention i.e. the sufficient water droplets-removing property, the durability such as abrasion resistance, weather resistance, etc. and economical efficiency.

Further, as a process for producing a substrate with a water repellent film in a case where in the substrate with a water repellent film of the present invention, the water repellent film further has an interlayer made mainly of silica between the substrate and the water repellent layer, a process may be mentioned which comprises a step of applying the composition for forming an interlayer on the surface of a substrate, followed by curing to form an interlayer made mainly of silica, and a step of applying the composition for forming a water repellent film of the present invention to the surface of the interlayer, followed by curing to form a water repellent layer.

Further, it is also possible to carry out curing treatment to form an interlayer and curing treatment to form a water repellent layer at the same time, by applying the composition for forming an interlayer to the surface of a substrate, followed by holding for a prescribed time to form a coating film, then applying the composition for forming a water repellent film to its surface to form a coating film, followed by curing treatment under a suitable condition.

The water repellent film for the substrate with a water repellent film of the present invention thus obtained, is one having a sufficient water droplets-removing property and durability such as abrasion resistance, weather resistance.

Here, in the water repellent film of the substrate with a water repellent film of the present invention, as described above, molecular structures of fluoroalkylene type carbon (-CF₂-) and fluoroether type carbon (-CF₂O-) are both contained in the material constituting the film. The ratio of the fluoroether type carbon to the fluoroalkylene type carbon in such a water repellent film can be represented by a peak ratio of [-CF₂O-]/[-CF₂-] as measured by an X-ray photoelectron spectrometer, for example, at the surface of the water repellent film. In the water repellent film of the substrate with a water repellent film of the present invention, the peak ratio of [-CF₂O-]/[-CF₂-] as measured by the X-ray photoelectron spectrometer, is preferably from 0.1 to 100, more preferably from 0.1 to 5.0.

Here, the -CF₂- structure in the water repellent film appears as a peak in the vicinity of 291.5 eV at the time of measuring C1sNarrow spectrum at the surface of the water repellent film by means of the X-ray photoelectron spectrometer. Likewise, the -CF₂O- structure appears as a peak in the vicinity of 293 eV in the C1sNarrow spectrum. Accordingly, the peak ratio of [-CF₂O-]/[-CF₂-] is obtained in such a manner that with respect to the surface of the water repellent film of a sample, C1sNarrow spectrum is measured by means of an X-ray photoelectron spectrometer, and from the obtained C1sNarrow spectrum, a peak in the vicinity of 291.5 eV attributable to -CF₂- and a peak in the vicinity of 293 eV attributable to -CF₂O- are separated usually by a curve fit function of the X-ray photoelectron spectrometer, to obtain the heights of the respect peaks, whereupon the peak ratio can be calculated from such values as [the height of the peak in the vicinity of 293 eV attributable to -CF₂O-]/[the height of the peak in the vicinity of 291.5 eV attributable to -CF₂-].

Further, the water droplets-removing property at the surface of the water repellent film of the substrate with a water repellent film of the present invention means such a property that deposition of water droplets is little and removal of deposited water droplets is easy, and for example, it can be evaluated from a peak ratio of F1s/Si2p as measured by an X-ray photoelectron spectrometry (ESCA), or by surface roughness Ra as measured by a scanning probe microscope (SPM).

The peak ratio of F1 s/Si2p at the surface of the water repellent film as measured by the X-ray photoelectron spectrometry (ESCA), is used as an index showing the proportions of fluorine atoms and silicon atoms present at the surface of the water repellent film, and it is meant that the larger the value of the peak ratio of F1s/Si2p, the higher the proportion of fluorine atoms at the surface of the water repellent film, and the better the water droplets-removing property. At the surface of the water repellent film of the substrate with a water repellent film of the present invention, the peak ratio of F1s/Si2p as measured by the X-ray photoelectron spectrometry (ESCA) is preferably from 1.5 to 7.0, more preferably from 2.0 to 6.0.

Further, with respect to the surface roughness (Ra) at the surface of the water repellent film as measured by the scanning probe microscope (SPM), it is meant that as this value decreases, deposition of water on the surface of the water repellent film becomes less likely to occur, and removal of water becomes easy. At the surface of the water repellent film of the substrate with a water repellent film of the present invention, such Ra is preferably from 0.1 to 5.0 nm, more preferably from 0.1 to 2.0 nm.

Further, at the surface of the water repellent film of the substrate with a water repellent film of the present invention, it is more preferred that both of the above peak ratio of F1s/Si2p and the above surface roughness Ra are within the above preferred ranges, i.e. the peak ratio of F1s/Si2p is from 1.5 to 7.0, and the surface roughness (Ra) is from 0.1 to 5.0 nm.

### <Article for transport equipment>

The substrate with a water repellent film of the present invention is suitably used in an application as an article for a transport equipment. The article for a transport equipment may, for example, be a body, window glass (front glass, side glass or rear glass), a mirror, a bumper, etc., in electric cars, automobiles, ships, or aircrafts.

With the substrate with a water repellent film of the present invention or the article for a transport equipment comprising such a substrate, its water repellent film surface has an excellent water droplets-removing property, whereby deposition of water droplets on the surface is little, and deposited water droplets will be readily repelled. In addition, by an interaction with wind pressure during transportation of the transport equipment, deposited water droplets rapidly move on the surface and will not stay as water droplets. Therefore, adverse effects to be brought about by moisture can be avoided. Further, the above water repellent film is excellent also in the abrasion resistance and weather resistance, and for example, this water droplets-removing property can be maintained even in its use as an article for a transport equipment for a long period of time under various conditions including its use outdoors.

With the substrate with a water repellent film of the present invention or the article for a transport equipment comprising such a substrate, particularly in an application in a sea-through field of e.g. various window glasses, it becomes very easy to secure a visual field by removal of water droplets, and the safety can be improved in the operation of trains, vehicles, etc. Further, in an environment where water droplets are likely to be frozen, freezing is less likely to occur, and even if frozen, thawing is quick. Further, deposition of water droplets scarcely occurs, whereby the number of cleaning operations can be reduced, and the cleaning operation can easily be carried out.

### EXAMPLES

Now, the present invention will be described with reference to Examples, but it should be understood that the present invention is by no means restricted by these Examples.

In each of the following Examples, evaluation of the substrate with a water repellent film was carried out as follows.

### <Water droplets-removing property>

The water droplets-removing property was evaluated by values of a water contact angle (CA) and a sliding angle of water (SA) as measured by the following methods. Firstly, before carrying out each of the following tests, the initial value was measured. Here, when the water contact angle (CA) is at least 90°, and the sliding angle of water (SA) is at most 20°, it can be said that such a substrate has a practically sufficient water droplets-removing property.

### [Water contact angle (CA)]

The contact angle of a water droplet having a diameter of 1 mm placed on the surface of a water repellent film of a substrate with a water repellent film, was measured by means of CA-X150 (manufactured by Kyowa Interface Science Co., Ltd.). Measurements were carried out at five different positions on the surface of the water repellent film, and the average value was calculated.

### [Sliding angle of water (SA)]

On the surface of a water repellent film of a substrate with a water repellent film held horizontally, a water droplet of 50 µL was dropped, and then, the substrate was gradually inclined, whereby the angle (sliding angle) between the horizontal plane and the substrate with a water repellent film at the time when the water droplet started sliding down, was measured by means of SA-11 (manufactured by Kyowa Interface Science Co., Ltd.). The smaller the sliding angle, the better the water droplets-removing property.

### <Abrasion resistance>

Against the surface of a water repellent film of a substrate with a water repellent film, an abrasion test was carried out under the following test conditions, whereupon the water contact angle and the sliding angle of water were measured by the above-described methods.

### [Cloth abrasion resistance test]

In accordance with JIS L0849, a cloth abrasion resistance test was carried out under the following test conditions by means of the following tester.
Tester: Reciprocating traverse tester (manufactured by KNT)
Test conditions: Cotton cloth (in accordance with JIS L0803), load: 1 kg,
number of abrasion times: 300 reciprocations

### [Door abrasion resistance test]

A door abrasion resistance test was carried out under the following test conditions by means of a door abrasion tester.

Test conditions: Wiping with a weather strip under a load of 1 N/cm² while spraying muddy water (spraying muddy water containing 5 mass% of one type of a test powder disclosed in JIS Z8901 on the surface of a test sample at 5.5 mL/m²·hr), the number of abrasion times: 4,000 reciprocations.

### <Weather resistance>

### [Outdoor exposure test]

In accordance with JIS Z2381, an outdoor exposure test was carried out. That is, a substrate with a water repellent film was set outdoors so that the surface of the water repellent film faced south at an angle of 30° to the horizontal plane, and upon expiration of three months from the initiation of the test, the water contact angle and the sliding angle of water were measured by the above-mentioned methods.

### [SWOM (Sunshine Weather-o-Meter) test]

In accordance with JIS D0205, a SWOM test was carried out. That is, the surface of a water repellent film of a substrate with a water repellent film was irradiated with ultraviolet rays for 1,500 hours, whereupon the water contact angle and the sliding angle of water were measured by the above-mentioned methods.

### [Evaluation of durability of water droplets-removing property]

With respect to the above-described four tests on the abrasion resistance and weather resistance, a case where all of the water contact angles (CA) after the tests are at least 90°, and all of the sliding angles of water (SA) were at most 20°, was judged to be acceptable "○", and a case other than such a case was judged to be unacceptable "×".

### <Surface properties of water repellent film>

In order to evaluate the properties of the water repellent film surface of a substrate with a water repellent film, the ratio of the fluoroether type carbon to the fluoroalkylene type carbon (-CF₂O-/-CF₂-), the ratio of fluorine atoms to silicon atoms (F1s/Si2p) and the surface roughness (Ra) were measured by the following methods.

### [-CF₂O-/-CF₂-]

With respect to water repellent film surfaces of the substrate with a water repellent films in Examples 54 to 57 prepared in the following Comparative Examples, as standard samples, Narrow spectrum analyses of C1s by X-ray (AIK α line) were carried out by means of an X-ray photoelectron spectrometer (XPS, Quantum 2000, manufactured by ULVAC-PHI, Inc.). From the results of the spectrum measurements, it was confirmed that a peak attributable to -CF₂O- appears in the vicinity of 293 eV, a peak attributable to CF₃- appears in the vicinity of 294 eV, and a peak attributable to -CF₂- appears in the vicinity of 291.5 eV.

Then, with respect to the water repellent film surface of a substrate with a water repellent film obtained in each Example, a Narrow spectrum analysis was carried out by means of the same X-ray photoelectron spectrometer as mentioned above. By utilizing the curve fit function of the X-ray photoelectron spectrometer based on the Narrow spectra of C1s of the above standard samples, the obtained Narrow spectrum of C1s in each Example was separated into a peak attributable to -CF₂O-, a peak attributable to -CF₂- and a peak attributable to CF₃-. By dividing the height of the peak attributable to -CF₂O- by the height of the peak attributable to -CF2-, -CF₂O-/-CF₂-was calculated.

### [F1s/Si2p]

With respect to the water repellent film surface of a substrate with a water repellent film, the measurement by X-ray (AIK α line) was carried out by means of a scanning X-ray photoelectron spectrometer (ESCA, Quantera SXM, manufactured by ULVAC-PHI, Inc.), whereupon the ratio of the height of the peak of F1s to the height of the peak of Si2p was calculated as F1s/Si2p.

### [Ra]

With respect to the water repellent film surface of a substrate with a water repellent film, the surface roughness (Ra) was measured by means of a scanning probe microscope (SPM, SPA-400, manufactured by SII Nano Technology Inc.) in DFM mode at a measuring area of 4 µm.

Further, abbreviations of compounds used in the respective Examples are shown below.

### <Compound (A)>

Comp. A1: C₈F₁₇C₂H₄Si(OCH₃)₃ (manufactured by SynQuest Laboratories, Inc.)
Comp. A2: C₆F₁₃C₂H₄Si(OCH₃)₃ (manufactured by SynQuest Laboratories, Inc.)
Comp. A3: C₄F₉C₂H₄Si(OCH₃)₃ (manufactured by Gelest, Inc.)
A4: C₄F₉CONHC₃H₆Si(OCH₃)₃
Comp. A5: C₆F₁₃C₂H₄Si(NCO)₃
Comp. A6: C₆F₁₃C₂H₄SiCl₃ (manufactured by SynQuest Laboratories, Inc.)

Here, with respect to the compounds (A1) to (A3) and (A6), commercial products were used, and with respect to the compounds (A4) and (A5), compounds obtained by the following Preparation Examples were, respectively, used. Confirmation of A4 and A5 obtained in Preparation Examples was carried out by 1H-NMR.

### [Preparation Example for compound (A4)]

Into a glass container equipped with a stirrer and a thermometer, 50.00 g of C₄F₉CO₂(C₂H₅) (manufactured by SynQuest Laboratories, Inc.) and 30.69 g of NH₂C₃H₆Si(OCH₃)₃ (KBM 903, trade name, manufactured by Shin-Etsu Chemical Co., Ltd.) were put and stirred for 12 hours at 25°C. Thereafter, unreacted compounds and byproduct ethanol were distilled off by distillation under reduced pressure to obtain 79.2 g of A4 which was liquid at room temperature.

### [Preparation Example of compound (A5)]

Based on a reference literature (Journal of Fluorine Chemistry 79 (1996) 87-91), 21.5 g of C₆F₁₃C2H₄SiCl₃ and 25.0 g of silver cyanate were used as starting materials and stirred in a benzene solvent at 80°C for one hour to prepare A5, which was purified to obtain 17.3 g of A5 which was liquid at room temperature.

### <Compound (B)>

B11: CF₃O[CF₂CF₂O]ₐCF₂CONHC₃H₆Si(OCH₃)₃
B12: CF₃O[CF₂CF₂O]ₐCF₂CH₂OCONHC₃H₆Si(OCH₃)₃
Comp. B13: CF₃O[CF₂CF₂O]ₐCF₂CH₂OC₃H₆Si(OCH₃)₃
(In all of the above B11, B12 and B13, a=7 to 8, average value: 7.3.)

Here, with respect to the above compounds (B11) to (B13), compounds obtained by the following Preparation Examples were, respectively, used. Confirmation of B11, B12 and B13 obtained in the Preparation Examples was carried out by 1H-NMR. Here, abbreviations for the compounds used in the Preparation Examples, are as follows.
R-225: dichloropentafluoropropane
R^{f}: -CF(CF₃)OCF₂CF(CF₃)OCF₂CF₂CF₃
R-113: CCl₂FCClF₂
CFE-419: CClF₂CClFCF₂OCF₂CF₂Cl
DBTDL: dibutyltin dilaurate

### [Preparation Example for compound (B11)]

Into a flask, 25 g of CH₃O[CH₂CH₂O]ₐCH₂CH₂OH (commercially available polyoxyethylene glycol monomethyl ether, a=7 to 8, average value: 7.3), 20 g of R-225, 1.2 g of NaF and 1.6 g of pyridine were put and vigorously stirred while maintaining the internal temperature to be at most 10°C and bubbling nitrogen. Into the flask, 46.6 g of FC(O)-R^{f} was dropwise added over a period of 3.0 hours while maintaining the temperature in the flask to be at most 5°C. After completion of the dropwise addition, stirring was continued at 50°C for 12 hours and further at room temperature for 24 hours, whereupon a reaction crude liquid was recovered. The recovered reaction crude liquid was subjected to filtration under reduced pressure, whereupon the filtrate was dried for 12 hours in a vacuum drier (50°C, 5.0 torr) to obtain a crude liquid. The crude liquid was dissolved in 100 mL of R-225 and washed three times with 1,000 mL of a saturated aqueous sodium bicarbonate solution to recover an organic phase. To the organic phase, 1.0 g of magnesium sulfate was added, followed by stirring for 12 hours, whereupon magnesium sulfate was removed by pressure filtration. Thereafter, from the filtrate, R-225 was distilled off by an evaporator to obtain 56.1 g of a compound (CH₃O[CH₂CH₂O]ₐCH₂CH₂OC(O)-R^{f} (a=7 to 8, average value: 7.3)) which was liquid at room temperature.

Then, into a 3,000 mL autoclave made of hastelloy, 1,560 g of R-113 was put and stirred, and maintained at 25°C. At the gas outlet of the autoclave, a condenser held at 20°C, a NaF pellet-packed layer and a condenser held at -20°C were set in series. Further, a liquid-returning line was set to return a liquid condensed in the condenser held at -20°C to the autoclave. After blowing nitrogen gas into the autoclave for 1.0 hour, fluorine gas diluted to 10 wt% with nitrogen gas (hereinafter referred to as 10% fluorine gas) was blown into at a flow rate of 24.8 L/hr for one hour. Then, into the autoclave, a solution having 27.5 g of CH₃O[CH₂CH₂O]ₐCH₂CH₂OC(O)-R^{f} dissolved in 1,350 g of R-113 was injected over a period f 30 hours, while blowing 10% fluorine gas at the same flow rate. Then, into the autoclave, 12 mL of R-113 was injected, while blowing 10% fluorine gas at the same flow rate. At that time, the internal temperature of the autoclave was changed to 40°C. Then, 6 mL of a R-113 solution having 1 mass% of benzene dissolved, was injected. Further, 10% fluorine gas was blown into for 1.0 hour, and then nitrogen gas was blown into for 1.0 hour. After completion of the reaction, the solvent was distilled off by vacuum drying (60°C for 6.0 hours), to obtain 45.4 g of a compound (CF₃O[CF₂CF₂O]ₐCF₂CF₂OC(O)-R^{f} (a=7 to 8, average value: 7.3)) which was liquid at room temperature.

Then, a 300 mL eggplant flask having a stirrer chip introduced, was thoroughly replaced with nitrogen. After replacement with nitrogen, into the eggplant flask, 40 g of ethanol, 5.6 g of NaF and 50 g of R-225 were put. Thereafter, into the eggplant flask, 43.5 g of CF₃O[CF₂CF₂O]ₐCF₂CF₂OC(O)-R^{f} was dropwise added and then vigorously stirred while bubbling with nitrogen at room temperature. The outlet of the eggplant flask was maintained to be a nitrogen atmosphere. After the reaction for 8 hours, while maintaining the interior of the system under reduced pressure by installing a vacuum pump on a cooling pipe, excess ethanol and CH₃CH₂OC(O)-R^{f} formed by the reaction were distilled off. After 24 hours, 26.8 g of a compound (CF₃O[CF₂CF₂O]ₐCF₂C(O)OCH₂CH₃ (a=7 to 8, average value: 7.3)) which was liquid at room temperature, was obtained.

Then, into a 100 mL round-bottomed flask, 33.1 g of

CF₃O[CF₂CF₂O]ₐCF₂C(O)OCH₂CH₃ and 3.7 g of NH₂CH₂CH₂CH₂Si(OCH₃)₃ were put and stirred at room temperature for 2 hours. After completion of the reaction, unreacted NH₂CH₂CH₂CH₂Si(OCH₃)₃ and byproduct ethanol were distilled off under reduced pressure to obtain 32.3 g of the compound (B11) which was liquid at room temperature.

### [Preparation Example for compound (B12)]

A 300 mL eggplant flask having a stirrer chip introduced, was thoroughly replaced with nitrogen. Then, into the eggplant flask, 30 g of 2-propanol, 50.0 g of R-225 and 4.1 g of NaBH₄ were put. The outlet of the eggplant flask was maintained to be a nitrogen atmosphere. Then, into the above eggplant flask, in the same manner as in the method for producing compound (B11), 26.2 g of obtained CF₃O[CF₂CF₂O]ₐCF₂C(O)OCH₂CH₃ (a=7 to 8, average value: 7.3) diluted with 30 g of R-225 was dropped, followed by vigorous stirring at room temperature. After the reaction for 8 hours, while maintaining the interior of the system to be under reduced pressure by installing a vacuum pump on a cooling pipe, the solvent was distilled off. After 24 hours, 100 g of R-225 was put into the eggplant flask, and while stirring, 500 g of a 0.2 N hydrochloric acid aqueous solution was dropwise added. After the dropwise addition, the reaction solution was stirred for 6 hours. Then, the reaction solution was separated into an aqueous phase and an organic phase, and the organic phase was washed three times with 500 g of distilled water, and by separation into two layers, an organic phase was recovered. Thereafter, 1.0 g of magnesium sulfate was added to the organic phase, followed by stirring for 12 hours. Thereafter, magnesium sulfate was removed by pressure filtration, and from the filtrate, R-225 was distilled off by an evaporator, to obtain 24.8 g of a compound (CF₃O[CF₂CF₂O]ₐCF₂CH₂OH (a=7 to 8, average value: 7.3)) which was liquid at room temperature.

Then, into a 250 mL round-bottomed flask, a solution having 10 g of CF₃O[CF₂CF₂O]ₐCF₂CH₂OH and 0.03 g of DBTDL as a catalyst dissolved in 25.0 g of CFE-419, was put in a nitrogen atmosphere, and the internal temperature was maintained to be at most 5°C. Into the round-bottomed flask, a solution having 0.3 g of commercially available (O)C=NCH₂CH₂CH₂Si(OCH₃)₃ dissolved in 10.0 g of CFE-419 was slowly dropwise added over a period of one hour, followed by stirring for about 12 hours. Then, excess (O)C=NCH₂CH₂CH₂Si(OCH₃) and CFE-419 were distilled off under reduced pressure to obtain 10.70 g of the compound (B12) which is liquid at room temperature.

### [Preparation Example for compound (B13)]

Into a 250 mL round-bottomed flask, 0.36 g of sodium hydride was put in a nitrogen atmosphere and washed with 25 mL of hexane, whereupon hexane was recovered. Such an operation was further repeated twice, whereupon remaining hexane was distilled off under reduced pressure. Then, into the round-bottomed flask, 25.0 g of CFE-419 was put, and the internal temperature was maintained to be at most 5°C. Then, a solution having 10 g of CF₃O[CF₂CF₂O]ₐCF₂CH₂OH (a=7 to 8, average value: 7.3) used also in the production of the above compound (B12) dissolved in 25.0 g of CFE-419, was slowly dropwise added over a period of one hour, followed by stirring for about 10 hours. Then, into the round-bottomed flask, 0.85 g of CClH₂CH₂CH₂Si(OCH₃)₃ was dropwise added and stirred at room temperature for two hours, followed by heating and refluxing for 72 hours. After completion of the heating and refluxing, the system was cooled to room temperature, and unreacted sodium hydride and byproduct sodium chloride were removed by pressure filtration, and from the filtrate, CFE-419 and excess CClH₂CH₂CH₂Si(OCH₃)₃ were distilled off under reduced pressure to obtain 10.70 g of the compound (B13) which is liquid at room temperature.

### <Compound (C)>

C1: Si(NCO)₄ (SI-400, manufactured by Matsumoto Pharmaceutical Manufacture Co., Ltd.)
C2: Si(OC₂H₅)₄ (manufactured by Aldrich)

As the above compounds (C1) and C2), commercial products were used.

### <1> Production of composition (H) for forming water repellent film

Now, Examples for preparation of the composition for forming a water repellent film of the present invention will be given. Examples 1 to 17 are working Examples of the present invention, and Examples 18 to 21 are Comparative Examples. A liquid composition (H) obtained in each Example was used for forming a water repellent layer in the subsequent Examples for producing a substrate with a water repellent film.

### Ref. [EXAMPLE 1]

Into a glass container equipped with a stirrer and a thermometer, 8.27 g of isopropyl alcohol (manufactured by Junsei Chemical Co., Ltd.), 7.24 g of hydrofluoroether (AE3000, manufactured by Asahi Glass Company, Limited), 0.34 g of compound (A1) and 0.74 g of compound (B11) were put and stirred at 25°C for 30 minutes. Then, 0.07 g of a 10 mass% nitric acid aqueous solution was added, followed by stirring at 25°C for two hours to obtain a liquid composition (H1) as a composition for forming a water repellent film.

### Ref. [EXAMPLE 2]

Into a glass container equipped with a stirrer and a thermometer, 10.34 g of isopropyl alcohol (manufactured by Junsei Chemical Co., Ltd.), 1.02 g of hydrofluoroether (AE3000, manufactured by Asahi Glass Company, Limited), 1.06 g of compound (A2) and 0.12 g of compound (B11) were put and stirred at 25°C for 30 minutes. Then, 0.13 g of a 10 mass% nitric acid aqueous solution was added, followed by stirring at 25°C for two hours to obtain a liquid composition (H2) as a composition for forming a water repellent film.

### Ref. [EXAMPLE 3]

Into a glass container equipped with a stirrer and a thermometer, 8.26 g of isopropyl alcohol (manufactured by Junsei Chemical Co., Ltd.), 7.23 g of hydrofluoroether (AE3000, manufactured by Asahi Glass Company, Limited), 0.35 g of compound (A2) and 0.74 g of compound (B11) were put and stirred at 25°C for 30 minutes. Then, 0.08 g of a 10 mass% nitric acid aqueous solution was added, followed by stirring at 25°C for two hours to obtain a liquid composition (H3) as a composition for forming a water repellent film.

### Ref. [EXAMPLE 4]

Into a glass container equipped with a stirrer and a thermometer, 6.21 g of isopropyl alcohol (manufactured by Junsei Chemical Co., Ltd.), 9.32 g of hydrofluoroether (AE3000, manufactured by Asahi Glass Company, Limited), 0.12 g of compound (A2) and 0.96 g of compound (B11) were put and stirred at 25°C for 30 minutes. Then, 0.06 g of a 10 mass% nitric acid aqueous solution was added, followed by stirring at 25°C for two hours to obtain a liquid composition (H4) as a composition for forming a water repellent film.

### Ref. [EXAMPLE 5]

Into a glass container equipped with a stirrer and a thermometer, 10.30 g of isopropyl alcohol (manufactured by Junsei Chemical Co., Ltd.), 5.15 g of hydrofluoroether (AE3000, manufactured by Asahi Glass Company, Limited), 0.59 g of compound (A2) and 0.53 g of compound (B11) were put and stirred at 25°C for 30 minutes. Then, 0.09 g of a 10 mass% nitric acid aqueous solution was added, followed by stirring at 25°C for two hours to obtain a liquid composition (H5) as a composition for forming a water repellent film.

### Ref. [EXAMPLE 6]

Into a glass container equipped with a stirrer and a thermometer, 12.40 g of isopropyl alcohol (manufactured by Junsei Chemical Co., Ltd.), 3.10 g of hydrofluoroether (AE3000, manufactured by Asahi Glass Company, Limited), 0.74 g of compound (A2) and 0.32 g of compound (B11) were put and stirred at 25°C for 30 minutes. Then, 0.10 g of a 10 mass% nitric acid aqueous solution was added, followed by stirring at 25°C for two hours to obtain a liquid composition (H6) as a composition for forming a water repellent film.

### Ref. [EXAMPLE 7]

Into a glass container equipped with a stirrer and a thermometer, 10.30 g of isopropyl alcohol (manufactured by Junsei Chemical Co., Ltd.), 5.15 g of hydrofluoroether (AE3000, manufactured by Asahi Glass Company, Limited), 0.59 g of compound (A2) and 0.53 g of compound (B12) were put and stirred at 25°C for 30 minutes. Then, 0.09 g of a 10 mass% nitric acid aqueous solution was added, followed by stirring at 25°C for two hours to obtain a liquid composition (H7) as a composition for forming a water repellent film.

### Ref. [EXAMPLE 8]

Into a glass container equipped with a stirrer and a thermometer, 10.30 g of isopropyl alcohol (manufactured by Junsei Chemical Co., Ltd.), 5.15 g of hydrofluoroether (AE3000, manufactured by Asahi Glass Company, Limited), 0.59 g of compound (A2) and 0.53 g of compound (B13) were put and stirred at 25°C for 30 minutes. Then, 0.09 g of a 10 mass% nitric acid aqueous solution was added, followed by stirring at 25°C for two hours to obtain a liquid composition (H8) as a composition for forming a water repellent film.

### Ref. [EXAMPLE 9]

Into a glass container equipped with a stirrer and a thermometer, 10.27 g of isopropyl alcohol (manufactured by Junsei Chemical Co., Ltd.), 5.13 g of hydrofluoroether (AE3000, manufactured by Asahi Glass Company, Limited), 0.62 g of compound (A3) and 0.53 g of compound (B11) were put and stirred at 25°C for 30 minutes. Then, 0.12 g of a 10 mass% nitric acid aqueous solution was added, followed by stirring at 25°C for two hours to obtain a liquid composition (H9) as a composition for forming a water repellent film.

### [EXAMPLE 10]

Into a glass container equipped with a stirrer and a thermometer, 8.26 g of isopropyl alcohol (manufactured by Junsei Chemical Co., Ltd.), 7.23 g of hydrofluoroether (AE3000, manufactured by Asahi Glass Company, Limited), 0.36 g of compound (A4) and 0.74 g of compound (B11) were put and stirred at 25°C for 30 minutes. Then, 0.08 g of a 10 mass% nitric acid aqueous solution was added, followed by stirring at 25°C for two hours to obtain a liquid composition (H10) as a composition for forming a water repellent film.

### Ref. [EXAMPLE 11]

Into a glass container equipped with a stirrer and a thermometer, 10.32 g of isopropyl alcohol (manufactured by Junsei Chemical Co., Ltd.), 5.16 g of hydrofluoroether (AE3000, manufactured by Asahi Glass Company, Limited), 0.57 g of compound (A1) and 0.53 g of compound (B11) were put and stirred at 25°C for 30 minutes. Then, 0.08 g of a 10 mass% nitric acid aqueous solution was added, followed by stirring at 25°C for two hours to obtain a liquid composition (H11) as a composition for forming a water repellent film.

### Ref. [EXAMPLE 12]

Into a glass container equipped with a stirrer and a thermometer, 0.31 g of butyl acetate (manufactured by Junsei Chemical Co., Ltd.), 12.38 g of hydrofluoroether (AE3000, manufactured by Asahi Glass Company, Limited), 1.08 g of compound (A6) and 0.12 g of compound (B11) were put and stirred at 25°C for 30 minutes to obtain a liquid composition (H12) as a composition for forming a water repellent film.

### Ref. [EXAMPLE 13]

Into a glass container equipped with a stirrer and a thermometer, 0.31 g of butyl acetate (manufactured by Junsei Chemical Co., Ltd.), 12.39 g of hydrofluoroether (AE3000, manufactured by Asahi Glass Company, Limited), 0.936 g of compound (A6) and 0.234 g of compound (B11) were put and stirred at 25°C for 30 minutes to obtain a liquid composition (H13) as a composition for forming a water repellent film.

### Ref. [EXAMPLE 14]

Into a glass container equipped with a stirrer and a thermometer, 0.31 g of butyl acetate (manufactured by Junsei Chemical Co., Ltd.), 12.40 g of hydrofluoroether (AE3000, manufactured by Asahi Glass Company, Limited), 0.812 g of compound (A6) and 0.348 g of compound (B11) were put and stirred at 25°C for 30 minutes to obtain a liquid composition (H14) as a composition for forming a water repellent film.

### Ref. [EXAMPLE 15]

Into a glass container equipped with a stirrer and a thermometer, 0.31 g of butyl acetate (manufactured by Junsei Chemical Co., Ltd.), 12.42 g of hydrofluoroether (AE3000, manufactured by Asahi Glass Company, Limited), 0.690 g of compound (A6) and 0.460 g of compound (B11) were put and stirred at 25°C for 30 minutes to obtain a liquid composition (H15) as a composition for forming a water repellent film.

### Ref. [EXAMPLE 16]

Into a glass container equipped with a stirrer and a thermometer, 0.31 g of butyl acetate (manufactured by Junsei Chemical Co., Ltd.), 12.43 g of hydrofluoroether (AE3000, manufactured by Asahi Glass Company, Limited), 0.565 g of compound (A6) and 0.565 g of compound (B11) were put and stirred at 25°C for 30 minutes to obtain a liquid composition (H16) as a composition for forming a water repellent film.

### Ref. [EXAMPLE 17]

Into a glass container equipped with a stirrer and a thermometer, 0.31 g of butyl acetate (manufactured by Junsei Chemical Co., Ltd.), 12.44 g of hydrofluoroether (AE3000, manufactured by Asahi Glass Company, Limited), 0.448 g of compound (A6) and 0.672 g of compound (B11) were put and stirred at 25°C for 30 minutes to obtain a liquid composition (H17) as a composition for forming a water repellent film.

### Ref. [EXAMPLE 18]

Into a glass container equipped with a stirrer and a thermometer, 5.18 g of isopropyl alcohol (manufactured by Junsei Chemical Co., Ltd.) and 10.37 g of hydrofluoroether (AE3000, manufactured by Asahi Glass Company, Limited) and 1.06 g of compound (B11) were put and stirred at 25°C for 30 minutes. Then, 0.05 g of a 10 mass% nitric acid aqueous solution was added, followed by stirring at 25°C for two hours to obtain a liquid composition (H18) as a composition for forming a water repellent film.

### Ref. [EXAMPLE 19]

Into a glass container equipped with a stirrer and a thermometer, 15.42 g of isopropyl alcohol (manufactured by Junsei Chemical Co., Ltd.) and 1.14 g of compound (A1) were put and stirred at 25°C for 30 minutes. Then, 0.11 g of a 10 mass% nitric acid aqueous solution was added, followed by stirring at 25°C for two hours to obtain a liquid composition (H19) as a composition for forming a water repellent film.

### Ref. [EXAMPLE 20]

Into a glass container equipped with a stirrer and a thermometer, 15.36 g of isopropyl alcohol (manufactured by Junsei Chemical Co., Ltd.) and 1.17 g of compound (A2) were put and stirred at 25°C for 30 minutes. Then, 0.14 g of a 10 mass% nitric acid aqueous solution was added, followed by stirring at 25°C for two hours to obtain a liquid composition (H20) as a composition for forming a water repellent film.

### Ref. [EXAMPLE 21]

Into a glass container equipped with a stirrer and a thermometer, 15.32 g of isopropyl alcohol (manufactured by Junsei Chemical Co., Ltd.) and 1.19 g of compound (A4) were put and stirred at 25°C for 30 minutes. Then, 0.15 g of a 10 mass% nitric acid aqueous solution was added, followed by stirring at 25°C for two hours to obtain a liquid composition (H21) as a composition for forming a water repellent film.

### <2> Formulation of liquid composition (E) for forming interlayer

Formulation Examples for the composition (E) for forming an interlayer used in the after-described Examples for production of a substrate with a water repellent film are shown below.

### [FORMULATION EXAMPLE 1]

Into a glass container equipped with a stirrer and a thermometer, 9.70 g of butyl acetate (manufactured by Junsei Chemical Co., Ltd.) and 0.30 g of compound (C1) were put and stirred at 25°C for 30 minutes to obtain a liquid composition (E1) for forming an interlayer.

### [FORMULATION EXAMPLE 2]

Into a glass container equipped with a stirrer and a thermometer, 97.00 g of butyl acetate (manufactured by Junsei Chemical Co., Ltd.), 2.85 g of compound (C1) and 0.15 g of compound (A5) were put and stirred at 25°C for 30 minutes to obtain a liquid composition (E2) for forming an interlayer.

### [FORMULATION EXAMPLE 3]

Into a glass container equipped with a stirrer and a thermometer, 9.70 g of butyl acetate (manufactured by Junsei Chemical Co., Ltd.), 0.24 g of compound (C1) and 0.06 g of compound (A5) were put and stirred at 25°C for 30 minutes to obtain a liquid composition (E3) for forming an interlayer.

### [FORMULATION EXAMPLE 4]

Into a glass container equipped with a stirrer and a thermometer, 9.70 g of isopropyl alcohol (manufactured by Junsei Chemical Co., Ltd.), 0.24 g of compound (C2), 0.06 g of compound (A2) and 0.27 g of a 10 mass% nitric acid aqueous solution were put and stirred at 25°C for one hour to obtain a liquid composition (E4) for forming an interlayer.

### [FORMULATION EXAMPLE 5]

Into a glass container equipped with a stirrer and a thermometer, 9.50 g of butyl acetate (manufactured by Junsei Chemical Co., Ltd.), 0.40 g of compound (C1) and 0.10 g of compound (A5) were put and stirred at 25°C for 30 minutes to obtain a liquid composition (E5) for forming an interlayer.

Here, liquid composition (E5) is the same in the blend components as the liquid composition (E3) formulated in the above Formulation Example 3, and the composition of the solid content (compounds (C1) and (A5)) is also the same, but the amount of the organic solvent is different. That is, in the liquid composition (E3), the amount of the organic solvent is 3,200 parts by mass per 100 parts by mass of the solid content, while in the liquid composition (E5), the amount of the organic solvent is 1,900 parts by mass per 100 parts by mass of the solid content.

### <3> Production and evaluation of substrate with a water repellent film

Using various liquid compositions obtained in the above respective Examples and Formulation Examples, water repellent films were formed on various substrates as described below to produce substrate with a water repellent films. With respect to the obtained substrate with a water repellent films, evaluations were carried out by the above-mentioned evaluation methods. The types of the substrate and liquid compositions used in the respective Examples are shown in Table 1, and the results obtained in the above respective evaluations are shown in Table 2.

### Ref. [EXAMPLES 22 to 24]

Using as a substrate a clean soda lime glass substrate polished with cerium oxide, washed and dried (water contact angle: 5°, 300 mm × 300 mm × thickness of 3 mm), for every Example shown in Table 1, 2 g of one of the liquid compositions H1 to H3 obtained in the above Examples 1 to 3 was applied on the surface of the glass substrate by a squeegee coating method. Then, it was held in a constant temperature and humidity tank set at 50°C under a relative humidity of 60% for 48 hours to form a water repellent layer thereby to obtain a substrate with a water repellent film having a water repellent film made of the water repellent layer.

### Ref. [EXAMPLES 25 to 32 and34, example 33

Using as a substrate a clean soda lime glass substrate having its surface polished with cerium oxide, washed and dried (water contact angle: 5°, 300 mm × 300 mm × thickness of 3 mm), for every Example shown in Table 1, 2 g of the liquid composition E1 obtained in the above Formulation Example 1 was applied on the surface of the glass substrate by a squeegee coating method and held at 25°C for one minute to form an interlayer. Then, for every Example shown in Table 1, 2 g of one of the liquid compositions H2 to H11 obtained in the above Examples 2 to 11 was applied to the surface of the formed interlayer by a squeegee coating method. Then, it was held in a constant temperature and humidity tank set at 50°C under a relative humidity of 60% for 48 hours to form a water repellent layer thereby to obtain a substrate with a water repellent film having a water repellent film composed of the interlayer and the water repellent layer.

### Ref. [EXAMPLES 35 to 37]

Using as a substrate a clean soda lime glass substrate having its surface polished with cerium oxide, washed and dried (water contact angle: 5°, 300 mm × 300 mm × thickness of 3 mm), for every Example shown in Table 1, 2 g of one of the liquid compositions E2 to E4 obtained in the above Formulation Examples 2 to 4 was applied on the surface of the glass substrate by a squeegee coating method and held at 25°C for one minute to form an interlayer. Then, for every Example shown in Table 1, 2 g of the liquid composition H5 obtained in the above Example 5 was applied on the surface of the formed interlayer by a squeegee coating method. Then, it was held in a constant temperature and humidity tank set at 50°C under a relative humidity of 60% for 48 hours to form a water repellent layer thereby to obtain a substrate with a water repellent film having a water repellent film composed of the interlayer and the water repellent layer.

### Ref. [EXAMPLE 38]

Using as a substrate a clean PET film having its surface washed with acetone and subjected to UV ozone gas treatment (water contact angle: 15°, 210 mm × 297 mm × thickness of 100 µm, HS-100, manufactured by Teijin DuPont Films Japan Limited), 2 g of the liquid composition E1 obtained in the above Formulation Example 1 on the surface of the PET film by a squeegee coating method and held at 25°C for one minute to obtain an interlayer. Then, 2 g of the liquid composition H5 obtained in the above Example 5 was applied on the surface of the formed interlayer by a squeegee coating method. Then, it was held in a constant temperature and humidity tank set at 50°C under a relative humidity of 60% for 48 hours to form a water repellent layer thereby to obtain a substrate with a water repellent film having a water repellent film composed of the interlayer and the water repellent layer.

### Ref. [EXAMPLES 39 to 41]

Using as a substrate a clean soda lime glass substrate having its surface polished with cerium oxide, washed and dried (water contact angle: 5°, 300 mm × 300 mm × thickness of 3 mm), for every Example shown in Table 1, 2 g of one of the liquid compositions E1, E2 and E3 obtained in the above Formulation Examples 1, 2 and 3 was applied on the surface of the glass substrate by a squeegee coating method and held at 25°C for one minute to form an interlayer. Then, for every Example shown in Table 1, 2 g of the liquid composition H5 obtained in the above Example 5 was applied on the surface of the formed interlayer by a squeegee coating method. Then, it was held at a constant temperature and humidity tank set at 25°C under a relative humidity of 50% for one hour to form a water repellent layer thereby to obtain a substrate with a water repellent film having a water repellent film composed of the interlayer and the water repellent layer.

### Ref. [EXAMPLES 42 to 47]

Using as a substrate, a clean soda lime glass substrate having its surface polished with cerium oxide, washed and dried (water contact angle: 5°, 300 mm × 300 mm × thickness of 3 mm), for every Example shown in Table 1, 2 g of the liquid composition E1 obtained in the above Formulation Example 1 was applied on the surface of the glass substrate by a squeegee coating method and held at 25°C for one minute to form an interlayer. Then, for every Example shown in Table 1, 2 g of one of the liquid compositions H12 to H17 obtained in the above Examples 12 to 17 was applied on the surface of the formed interlayer by a squeegee coating method. Then, it was held at a constant temperature and humidity tank set at 50°C under a relative humidity of 60% for 48 hours to form a water repellent layer thereby to obtain a substrate with a water repellent film having a water repellent film composed of the interlayer and the water repellent layer.

### Ref. [EXAMPLES 48 to 53]

Using as a substrate a clean soda lime glass substrate having its surface polished with cerium oxide, washed and dried (water contact angle: 5°, 300 mm × 300 mm × thickness of 3 mm), for every Example shown in Table 1, 2 g of the liquid composition E5 obtained in the above Formulation Example 5 was applied on the surface of the glass substrate by a squeegee coating method and held at 25°C for one minute to form an interlayer. Then, for every Example shown in Table 1, 2 g of one of the liquid compositions H12 to H17 obtained in the above Examples 12 to 17 was applied on the surface of the formed interlayer by a squeegee coating method. Then, it was held in a constant temperature and humidity tank set at 50°C under a relative humidity of 60% for 48 hours to form a water repellent layer thereby to obtain a substrate with a water repellent film having a water repellent film composed of the interlayer and the water repellent layer.

### Ref. [EXAMPLES 54 to 57]

Using as a substrate, a clean soda lime glass substrate having its surface polished with cerium oxide, washed and dried (water contact angle: 5°, 300 mm × 300 mm × thickness of 3 mm), for every Example shown in Table 1, 2 g of the liquid composition E1 obtained in the above Formulation Example 1 was applied on the surface of the glass substrate by a squeegee coating method and held at 25°C for one minute to form an interlayer. Then, for every Example shown in Table 1, 2 g of one of the liquid compositions H18 to H21 obtained in the above Examples 18 to 21 was applied on the surface of the formed interlayer by a squeegee coating method. Then, it was held in a constant temperature and humidity tank set at 50°C under a relative humidity of 60% for 48 hours to form a water repellent layer thereby to obtain a substrate with a water repellent film having a water repellent film composed of the interlayer and the water repellent layer.

**TABLE 1**

| Example | Materials for forming substrate with a water repellent film | | | | | | | | |
|---|---|---|---|---|---|---|---|---|---|
| | Substrate | Composition for forming water repellent film | | | | Composition for forming interlayer | | | |
| | | Composition | Compound (A) | Compound (B) | B/[A+B] [wt%] | Composition | Compound (A) | Compound (B) | A/[A+C] [wt%] |
| Ref. 22 | Glass | H1 | A1 | B11 | 69% | - | - | - | - |
| Ref. 23 | Glass | H2 | A2 | B11 | 10% | - | - | - | - |
| Ref. 24 | Glass | H3 | A2 | B11 | 68% | - | - | - | - |
| Ref. 25 | Glass | H4 | A2 | B11 | 89% | E1 | - | C1 | - |
| Ref. 26 | Glass | H3 | A2 | B11 | 68% | E1 | - | C1 | - |
| Ref. 27 | Glass | H5 | A2 | B11 | 47% | E1 | - | C1 | - |
| Ref. 28 | Glass | H6 | A2 | B11 | 30% | E1 | - | C1 | - |
| Ref. 29 | Glass | H2 | A2 | B11 | 10% | E1 | - | C1 | - |
| Ref. 30 | Glass | H7 | A2 | B12 | 47% | E1 | - | C1 | - |
| Ref. 31 | Glass | H8 | A2 | B13 | 47% | E1 | - | C1 | - |
| Ref. 32 | Glass | H9 | A3 | B11 | 46% | E1 | - | C1 | - |
| 33 | Glass | H10 | A4 | B11 | 67% | E1 | - | C1 | - |
| Ref. 34 | Glass | H11 | A1 | B11 | 48% | E1 | - | C1 | - |
| Ref. 35 | Glass | H5 | A2 | B11 | 47% | E2 | A5 | C1 | 5% |
| Ref. 36 | Glass | H5 | A2 | B11 | 47% | E3 | A5 | C1 | 20% |
| Ref. 37 | Glass | H5 | A2 | B11 | 47% | E4 | A² | C2 | 20% |
| Ref. 38 | PET | H5 | A2 | B11 | 47% | E1 | - | C1 | - |
| Ref. .39 | Glass | H5 | A2 | B11 | 47% | E1 | - | C1 | - |
| Ref. 40 | Glass | H5 | A2 | B11 | 47% | E2 | A5 | C1 | 5% |
| Ref. 41 | Glass | H5 | A2 | B11 | 47% | E3 | A5 | C1 | 20% |
| Ref. 42 | Glass | H12 | A6 | B11 | 10% | E1 | - | C1 | - |
| Ref. 43 | Glass | H13 | A6 | B11 | 20% | E1 | - | C1 | - |
| Ref. 44 | Glass | H14 | A6 | B11 | 30% | E1 | - | C1 | - |
| Ref. 45 | Glass | H15 | A6 | B11 | 40% | E1 | - | C1 | - |
| Ref. 46 | Glass | H16 | A6 | B11 | 50% | E1 | - | C1 | - |
| Ref. 47 | Glass | H17 | A6 | B11 | 60% | E1 | - | C1 | - |
| Ref. 48 | Glass | H12 | A6 | B11 | 10% | E5 | A5 | C1 | 20% |
| Ref. 49 | Glass | H13 | A6 | B11 | 20% | E5 | A5 | C1 | 20% |
| Ref. 50 | Glass | H14 | A6 | B11 | 30% | E5 | A5 | C1 | 20% |
| Ref. 51 | Glass | H15 | A6 | B11 | 40% | E5 | A5 | C1 | 20% |
| Ref. 52 | Glass | H16 | A6 | B11 | 50% | E5 | A5 | C1 | 20% |
| Ref. 53 | Glass | H17 | A6 | B11 | 60% | E5 | A5 | C1 | 20% |
| Ref. 54 | Glass | H18 | - | B11 | 100% | E1 | - | C1 | - |
| Ref. 55 | Glass | H19 | A1 | - | % | E1 | - | C1 | - |
| Ref. 56 | Glass | H20 | A2 | - | 0% | E1 | - | C1 | - |
| Ref. 57 | Glass | H21 | A4 | - | 0% | E1 | - | C1 | - |

**TABLE 2**

| Example | Surface properties of water repellent film | | | Evaluation of water droplets-removing property on water repellent film surface | | | | | | | | | | |
|---|---|---|---|---|---|---|---|---|---|---|---|---|---|---|
| | | | | Water contact angle (CA) [°] | | | | | Sliding angle of water (SA)[° ] | | | | | Evaluation of durability |
| | - CF₂O- / -CF₂- | F1s/ Si2p | Ra [nm] | Initial | Abrasion resistance | | Weather resistance | | Initial | Abrasion resistance | | Weather resistance | | |
| | | | | | Cloth | Door abrasion | Outdoor exposure | SWOM | | Cloth | Door abrasion | Outdoor exposure | SWOM | |
| Ref. 22 | 0.7 | 1.9 | 2.1 | 112 | 108 | 109 | 104 | 107 | 10 | 13 | 10 | 15 | 12 | ○ |
| Ref. 23 | 0.1 | 1.5 | 2.5 | 111 | 95 | 92 | 106 | 104 | 12 | 20 | 15 | 19 | 17 | ○ |
| Ref. 24 | 1.1 | 1.6 | 2.0 | 111 | 108 | 108 | 105 | 106 | 12 | 14 | 16 | 16 | 14 | ○ |
| Ref. 25 | 4.3 | 2.4 | 1.2 | 110 | 107 | 108 | 108 | 106 | 11 | 14 | 10 | 11 | 15 | ○ |
| Ref. 26 | 1.1 | 5.2 | 1.3 | 109 | 108 | 109 | 107 | 104 | 11 | 15 | 13 | 11 | 14 | ○ |
| Ref. 27 | 0.7 | 6.2 | 1.5 | 108 | 106 | 109 | 108 | 104 | 10 | 15 | 11 | 12 | 14 | ○ |
| Ref. 28 | 0.2 | 2.0 | 1.2 | 108 | 106 | 107 | 106 | 102 | 9 | 17 | 12 | 12 | 13 | ○ |
| Ref. 29 | 0.1 | 1.8 | 2.4 | 107 | 94 | 100 | 104 | 99 | 13 | 19 | 19 | 13 | 17 | ○ |
| Ref. 30 | 0.7 | 2.2 | 4.2 | 111 | 105 | 108 | 108 | 103 | 10 | 13 | 13 | 12 | 10 | ○ |
| 33 | 0.7 | 2.0 | 2.2 | 111 | 103 | 102 | 105 | 103 | 10 | 16 | 15 | 16 | 16 | ○ |
| Ref. 32 | 1.1 | 1.7 | 3.1 | 107 | 102 | 105 | 104 | 101 | 13 | 11 | 18 | 16 | 15 | ○ |
| Ref. 33 | 1.1 | 2.1 | 1.3 | 108 | 106 | 109 | 105 | 102 | 12 | 15 | 12 | 12 | 15 | ○ |
| Ref. 34 | 0.5 | 2.4 | 1.0 | 111 | 110 | 109 | 108 | 105 | 13 | 13 | 10 | 15 | 12 | ○ |
| Ref. 35 | 0.6 | 2.3 | 0.7 | 109 | 107 | 110 | 109 | 105 | 12 | 13 | 12 | 11 | 13 | ○ |
| Ref. 36 | 0.3 | 2.5 | 0.5 | 110 | 106 | 109 | 111 | 106 | 10 | 15 | 15 | 12 | 14 | ○ |
| Ref. 37 | 0.3 | 1.9 | 1.1 | 111 | 107 | 107 | 110 | 106 | 12 | 13 | 13 | 13 | 16 | ○ |
| Ref. 38 | 0.7 | - | - | 110 | 109 | - | 110 | 102 | 14 | 15 | - | 15 | 16 | ○ |
| Ref. 39 | 0.7 | 1.5 | 2.1 | 111 | 108 | 107 | 105 | 106 | 9 | 13 | 16 | 15 | 12 | ○ |
| Ref. 40 | 0.6 | 1.8 | 1.6 | 112 | 109 | 109 | 107 | 107 | 10 | 12 | 12 | 13 | 13 | ○ |
| Ref. 41 | 0.3 | 2.2 | 1.0 | 111 | 107 | 109 | 109 | 106 | 9 | 13 | 18 | 12 | 12 | ○ |
| Ref. 42 | 0.1 | 1.8 | 4.6 | 110 | 100 | 98 | 106 | 106 | 13 | 15 | 15 | 14 | 15 | ○ |
| Ref. 43 | 0.1 | 2.3 | 4.2 | 111 | 108 | 108 | 105 | 105 | 11 | 12 | 14 | 13 | 15 | ○ |
| Ref. 44 | 0.2 | 2.5 | 4.3 | 112 | 109 | 107 | 106 | 103 | 10 | 13 | 12 | 15 | 14 | ○ |
| Ref. 45 | 0.3 | 3.9 | 3.5 | 110 | 109 | 107 | 105 | 104 | 10 | 12 | 13 | 16 | 16 | ○ |
| Ref. 46 | 0.7 | 5.1 | 2.3 | 110 | 110 | 109 | 102 | 103 | 9 | 10 | 10 | 15 | 17 | ○ |
| Ref. 47 | 0.8 | 4.8 | 2.1 | 111 | 110 | 110 | 103 | 101 | 10 | 10 | 11 | 17 | 19 | ○ |
| Ref. 48 | 0.1 | 1.9 | 4.5 | 111 | 96 | 100 | 105 | 105 | 13 | 14 | 15 | 15 | 14 | ○ |
| Ref. 49 | 0.1 | 2.2 | 4.7 | 110 | 105 | 102 | 106 | 105 | 11 | 11 | 13 | 15 | 15 | ○ |
| Ref. 50 | 0.2 | 2.8 | 3.8 | 112 | 107 | 108 | 104 | 106 | 10 | 13 | 12 | 16 | 15 | ○ |
| Ref. 51 | 0.2 | 4.2 | 3.2 | 112 | 110 | 108 | 103 | 104 | 10 | 12 | 13 | 18 | 18 | ○ |
| Ref. 52 | 0.4 | 4.7 | 3.5 | 111 | 110 | 109 | 103 | 103 | 10 | 13 | 11 | 16 | 17 | ○ |
| Ref. 53 | 0.6 | 4.5 | 2.6 | 110 | 108 | 109 | 102 | 102 | 11 | 11 | 11 | 19 | 18 | ○ |
| Ref. 54 | (∞) | 2.5 | 1.0 | 111 | 112 | 108 | 100 | 106 | 11 | 12 | 10 | 23 | 15 | × |
| Ref. 55 | 0.0 | 2.8 | 3.2 | 108 | 100 | 99 | 108 | 106 | 14 | 17 | 21 | 13 | 18 | × |
| Ref. 56 | 0.0 | 2.5 | 3.0 | 109 | 47 | 95 | 106 | 102 | 14 | - | 18 | 14 | 19 | × |
| Ref. 57 | 0.0 | 2.0 | 2.4 | 105 | 34 | - | - | - | 16 | - | - | - | - | × |

| | | | | | | | | | | | | | | |
|---|---|---|---|---|---|---|---|---|---|---|---|---|---|---|
| * In Example 54, the results (∞) for (-CF₂O-/-CF₂-) is due to the fact that -CF₂- was "0". | | | | | | | | | | | | | | |

In Table 2, "-' in the evaluation results means that the measurement was impossible in Example 38 since the substrate was PET and in Examples 56 to 57 since the water contact angle was low. From the above Tables 1 and 2, it is evident that with respect to the substrate with a water repellent films in Examples 22 to 53 having water repellent layers formed by using the compositions for forming water repellent films of the present invention obtained in Examples 1 to 17, each of them is excellent in the water droplets-removing property from the initial stage to even after the durability test:

On the other hand, with respect to the substrate with a water repellent films in Examples 54 to 57 having water repellent layers formed by using the compositions for forming water repellent films in Comparative Examples wherein only either one of the compounds (A) and (B) obtained in Examples 18 to 21 was used, each of them has a problem with respect to the water droplets-removing property after the durability test, although there is no problem with respect to the initial water droplets-removing property.

### Ref [EXAMPLE 58]

Using as a substrate a clean door glass for an automobile having its surface polished with cerium oxide, washed and dried, the liquid compositions E1 to E3 obtained in the above Formulation Examples 1 to 3 were applied on the surface of the glass by a squeegee coating method and held at 25°C for one minute to form an interlayer. Then, the liquid composition H5 obtained in the above Example 5 was applied on the surface of the formed interlayer by a squeegee coating method. Then, it was held in an atmosphere at 25°C under a relative humidity of 50% for one hour to form a water repellent layer thereby to obtain a water repellent glass for an automobile having a water repellent film composed of the interlayer and the water repellent layer.

With respect to 20 positions on the water repellent film surface of the obtained door glass, the water contact angle and the sliding angle of water were measured by the above-described methods, whereby they were within ranges of from 110 to 112° and from 10 to 12°, respectively. Further, the thicknesses of the interlayer and the water repellent layer measured by a transmission electron microscope JEM-2010F (manufactured by JEOL Ltd.) were from 5 to 15 nm and from 10 to 15 nm, respectively.

Two sheets of this water repellent glass were mounted on the door of an automobile and subjected to a driving test at the time of raining, whereby on each water repellent glass, water droplets on the water repellent film surface were repelled, and the water droplets were readily moved by the interaction with the window pressure by driving, and the side viewing field was secured.

Further, driving tests for two hours per day were carried out for three months, and deposition state of water droplets on the water repellent film surface of the water repellent glass was visually observed, whereby on each water repellent glass, no substantial deposition of water droplets was observed.

### Ref [EXAMPLE 59]

By changing the door glass in the above Example 58 to a front laminated glass or a rear glass, a water repellent front laminated glass for an automobile and a water repellent rear glass for an automobile were obtained. These water repellent glasses for automobiles were mounted on automobiles, and the same driving tests as in Example 58 were carried out, whereby the same effects as in Example 58 were confirmed.

### Ref [EXAMPLE 60]

A door glass of an automobile which had been used for five years, was polished with calcium carbonate, then washed with water and dried for one hour. On this glass, a water repellent film was formed by the same method as the method in Example 58. By using this automobile, the running test was carried out in the same manner as in Example 58, whereby the same effects as in Example 58 were confirmed.

### INDUSTRIAL APPLICABILITY

With the substrate with a water repellent film of the present invention, its water repellent film surface has an excellent water droplets-removing property and is further excellent also in the abrasion resistance and weather resistance, and thus, it is suitable for an application as an article such as a body, a window glass (front glass, side glass or rear glass), a mirror or a bumper in transport equipments such as trains, automobiles, ships, and aircrafts.

## Claims

1. A composition for forming a water repellent film, the composition comprising
(I) a compound (A) and a compound (B), or
(II) a partially hydrolyzed co-condensate of:
(i) a compound represented by a formula (1 a) and/or a partially hydrolyzed condensate thereof; and
(ii) a compound represented by a formula (2a) and/or a partially hydrolyzed condensate thereof, wherein
the compound (A) is at least one fluorinated organic silicon compound containing no etheric oxygen atom and being selected from the group consisting of a compound represented by the following formula (1a), a partially hydrolyzed condensate thereof, and a compound represented by the following formula (1b),
R^{f1}-Y-Si(R¹¹)ᵣ(X¹)₃₋ᵣ (1a) 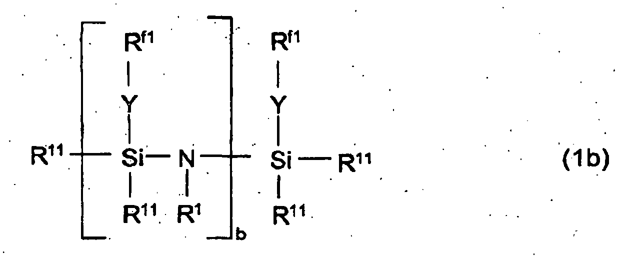
(where in the formulae (1a) and (1b), R^{f1} is a C₁₋₇ perfluoroalkyl group having the formula CF₃(CF₂)ₗ-, wherein I is an integer of from 0 to 6, which contains no etheric oxygen atom between carbon-carbon atoms, Y is a C₁₋₆ bivalent organic group which contains no fluorine atom, R¹¹ each independently is a hydrogen atom or a C₁-₆ hydrocarbon group which contains no fluorine atom, X¹ each independently is a halogen atom, an alkoxy group or an isocyanate group, r is an integer of from 0 to 2, R¹ is a hydrogen atom or a C₁₋₃ hydrocarbon group which contains no fluorine atom, and b is an integer of from 1 to 100), and wherein
the compound (B) is at least one fluorinated organic silicon compound containing an etheric oxygen atom and being selected from the group consisting of a compound represented by the following formula (2a), a partially hydrolyzed condensate thereof, and a compound represented by the following formula (2b),
R^{f2}-W-Z-Si(R¹²)ₚ(X²)₃₋ₚ (2a) 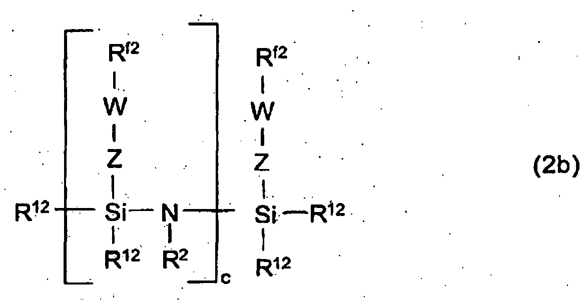
(where in the formulae (2a) and (2b), R^{f2} is a C₁₋₇ perfluoroalkyl group having the formula CF₃(CF₂)ₗ-, wherein I is an integer of from 0 to 6, which may have an etheric oxygen atom inserted between carbon-carbon atoms, W is -O-(CF₂CF₂O)ₐ-CF₂-(wherein a is an integer of from 1 to 200), Z is a bivalent organic group selected from -CONHC₃H₆- and -CH₂OCO-NHC₃H₆-, R¹² each independently is a hydrogen atom or a C₁₋₆ hydrocarbon group which contains no fluorine atom, X² each independently is a halogen atom, an alkoxy group or an isocyanate group, p is an integer of from 0 to 2, R² is a hydrogen atom or a C₁₋₃ hydrocarbon group which contains no fluorine atom, and c is an integer of from 1 to 100).

2. The composition for forming a water repellent film according to Claim 1, wherein the mass percentage of the compound (B) to the total mass of the compounds (A) and (B) represented by [compound (B)]/[compounds (A) and (B)] x 100 in the composition (provided that in a case where the composition contains a partially hydrolyzed co-condensate, with respect to the partially hydrolyzed co-condensate, the amounts of the compounds (A) and (B) before the hydrolytic co-condensation reaction, are used for the calculation of the mass percentage), is from 10 to 90 mass%.

3. A substrate with a water repellent film comprising a substrate and a water repellent film formed on at least a part of the surface of the substrate, wherein the water repellent film is composed of at least one layer and has, as the outermost layer, a water repellent layer formed by using the composition for forming a water repellent film as defined in Claim 1 or 2.

4. The substrate with a water repellent film according to Claim 3, wherein the water repellent film further has an interlayer made mainly of silica, between the substrate and the water repellent layer.

5. The substrate with a water repellent film according to Claim 4, wherein the interlayer is a layer formed by using a composition for forming an interlayer which contains at least one compound (C) selected from the group consisting of a compound represented by the following formula (3), its partially hydrolysed condensate, and a perhydropolysilazane,
Si(X³)₄ (3)
(in the formula (3), X³ each independently is a halogeh atom, an alkoxy group or an isocyanate group).

6. The substrate with a water repellent film according to Claim 5, wherein the composition for forming an interlayer further contains the compound (A) or contains, instead of the compound (C), a partially hydrolyzed co-condensate of the compound represented by the formula (3) and/or its partially hydrolyzed condensate, and the compound represented by the formula (1a) and/or its partially hydrolyzed condensate, and the mass percentage of the compound (A) to the total mass of the compounds (A) and (C) represented by [compound (A)]/[compounds (A) and (C)] x 100 in the composition (provided that in a case where the composition contains the partially hydrolyzed co-condensate, with respect to the partially hydrolyzed co-condensate, the amounts of the compounds (A) and (C) before the hydrolytic co-condensation
reaction, are used for the calculation of the mass percentage), is from 5 to 70 mass%.

7. The substrate with a water repellent film according to any one of Claims 3 to 6, wherein the peak ratio of [-CF₂O-]/[-CF₂-] as measured by an X-ray photoelectron spectrometer at the surface of the water repellent film is from 0.1 to 10.0.

8. The substrate with a water repellent film according to any one of Claims 3 to 7, wherein the peak ratio of F1s/Si2p as measured by an X-ray photoelectron spectroscopy (ESCA) at the surface of the water repellent film is from 1.5 to 7.0, and Ra (surface roughness) as measured by a scanning probe microscope (SPM) is from 0.1 to 5.0 nm.

9. A process for producing the substrate with a water repellent film as defined in any one of Claims 3 to 8, which comprises a step of applying the composition for forming a water repellent film as defined in Claim 1 or 2 to the surface of a substrate or to the surface of a layer to constitute a lower layer of the outermost layer in the water repellent film, preliminarily formed on the surface of a substrate, followed by curing to form a water repellent layer.

10. A process for producing the substrate with a water repellent film as defined in any one of Claims 4 to 8, which comprises a step of applying a composition for forming an interlayer to the surface of a substrate, followed by curing to form an interlayer made mainly of silica, and a step of applying the composition for forming a water repellent film as defined in Claim 1 or 2 to the surface of the interlayer, followed by curing to form a water repellent layer.

11. An article for a transport equipment, which comprises the substrate with a water repellent film as defined in any one of Claims 3 to 7.

## Patentansprüche

1. Zusammensetzung zur Bildung eines wasserabweisenden Films, wobei die Zusammensetzung
(I) eine Verbindung (A) und eine Verbindung (B) oder
(II) ein teilweise hydrolysiertes Cokondensat aus:
(i) einer Verbindung, die durch die Formel (1a) dargestellt ist und/oder einem teilweise hydrolysierten Kondensat davon, und
(ii) einer Verbindung, die durch die Formel (2a) dargestellt ist und/oder einem teilweise hydrolysierten Kondensat davon,
umfasst,
wobei die Verbindung (A) mindestens eine fluorierte organische Siliziumverbindung ist, die kein etherisches Sauerstoffatom enthält und aus der Gruppe, bestehend aus einer Verbindung, die durch die folgende Formel (1 a) dargestellt ist, einem teilweise hydrolysierten Kondensat davon und einer Verbindung, die durch die folgende Formel (1 b) dargestellt ist, ausgewählt ist,
R^{f1}-Y-Si(R¹¹)ᵣ(X1)₃₋ᵣ (1a) 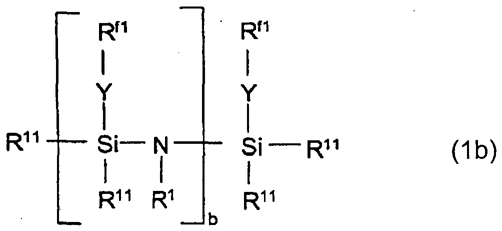
(wobei in den Formeln (1 a) und (1 b) R^{f1} eine C₁₋₇-Perfluoralkylgruppe mit der Formel CF₃(CF₂)ₗ- ist, worin I eine ganze Zahl von 0 bis 6 ist, die kein etherisches Sauerstoffatom zwischen Kohlenstoff-Kohlenstoff-Atomen enthält, Y eine zweiwertige organische C₁₋₆-Gruppe ist, die kein Fluoratom enthält, R¹¹ jeweils unabhängig ein Wasserstoffatom oder eine C₁₋₆-Kohlenwasserstoffgruppe ist, die kein Fluoratom enthält, X¹ jeweils unabhängig ein Halogenatom, eine Alkoxygruppe oder eine Isocyanatgruppe ist, r eine ganze Zahl von 0 bis 2 ist, R¹ ein Wasserstoffatom oder eine C₁₋₃-Kohlenwasserstoffgruppe ist, die kein Fluoratom enthält, und b eine ganze Zahl von 1 bis 100 ist), und wobei
die Verbindung (B) mindestens eine fluorierte organische Siliziumverbindung ist, die ein etherisches Sauerstoffatom enthält und aus der Gruppe, bestehend aus einer Verbindung, die durch die folgende Formel (2a) dargestellt ist, einem teilweise hydrolysierten Kondensat davon und einer Verbindung, die durch die folgende Formel (2b) dargestellt ist, ausgewählt ist,
R^{f2}-W-Z-Si(R¹²)ₚ(X²)₃₋ₚ (2a) 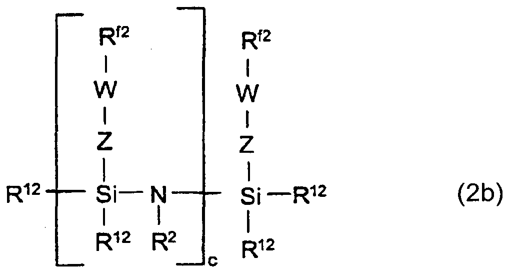
(wobei in den Formeln (2a) und (2b) R^{f2} eine C₁₋₇-Perfluoralkylgruppe mit der Formel CF₃(CF₂)ₗ- ist, worin I eine ganze Zahl von 0 bis 6 ist, die ein etherisches Sauerstoffatom aufweisen kann, das zwischen Kohlenstoff-Kohlenstoff-Atomen insertiert ist, W -O-(CF₂CF₂O)ₐ-CF₂- ist (worin a eine ganze Zahl von 1 bis 200 ist), Z eine zweiwertige organische Gruppe ist, die aus -CONHC₃H₆- und -CH₂OCO-NHC₃H₆- ausgewählt ist, R¹² jeweils unabhängig ein Wasserstoffatom oder eine C₁₋₆-Kohlenwasserstoffgruppe ist, die kein Fluoratom enthält, X² jeweils unabhängig ein Halogenatom, eine Alkoxygruppe oder eine Isocyanatgruppe ist, p eine ganze Zahl von 0 bis 2 ist, R² ein Wasserstoffatom oder eine C₁₋₃-Kohlenwasserstoffgruppe ist, die kein Fluoratom enthält, und c eine ganze Zahl von 1 bis 100 ist).

2. Zusammensetzung zur Bildung eines wasserabweisenden Films nach Anspruch 1, bei welcher der Massenprozentsatz der Verbindung (B) bezogen auf die Gesamtmasse der Verbindungen (A) und (B), der durch [Verbindung (B)]/[Verbindungen (A) und (B)] x 100 dargestellt ist, in der Zusammensetzung (mit der Maßgabe, dass in einem Fall, bei dem die Zusammensetzung ein teilweise hydrolysiertes Cokondensat enthält, bezogen auf das teilweise hydrolysierte Cokondensat die Mengen der Verbindungen (A) und (B) vor der hydrolytischen Cokondensationsreaktion für die Berechnung des Massenprozentsatzes verwendet werden) von 10 bis 90 Massen-% beträgt.

3. Substrat mit einem wasserabweisenden Film, das ein Substrat und einen wasserabweisenden Film umfasst, der auf mindestens einem Teil der Oberfläche des Substrats ausgebildet ist, wobei der wasserabweisende Film aus mindestens einer Schicht zusammengesetzt ist und als äußerste Schicht eine wasserabweisende Schicht aufweist, die unter Verwendung der Zusammensetzung zur Bildung eines wasserabweisenden Films nach Anspruch 1 oder 2 ausgebildet worden ist.

4. Substrat mit einem wasserabweisenden Film nach Anspruch 3, bei dem der wasserabweisende Film ferner eine Zwischenschicht, die vorwiegend aus Siliziumoxid hergestellt ist, zwischen dem Substrat und der wasserabweisenden Schicht aufweist.

5. Substrat mit einem wasserabweisenden Film nach Anspruch 4, bei dem die Zwischenschicht eine Schicht ist, die unter Verwendung einer Zusammensetzung zur Bildung einer Zwischenschicht ausgebildet worden ist, die mindestens eine Verbindung (C) enthält, die aus der Gruppe, bestehend aus einer Verbindung, die durch die folgende Formel (3) dargestellt ist, deren teilweise hydrolysiertem Kondensat und einem Perhydropolysilazan, ausgewählt ist,
Si(X³)₄ (3)
(in der Formel (3) ist X³ jeweils unabhängig ein Halogenatom, eine Alkoxygruppe oder eine Isocyanatgruppe).

6. Substrat mit einem wasserabweisenden Film nach Anspruch 5, bei dem die Zusammensetzung zur Bildung einer Zwischenschicht ferner die Verbindung (A) enthält oder anstelle der Verbindung (C) ein teilweise hydrolysiertes Cokondensat der Verbindung, die durch die Formel (3) dargestellt ist, und/oder deren teilweise hydrolysiertem Kondensat und der Verbindung, die durch die Formel (1 a) dargestellt ist, und/oder deren teilweise hydrolysiertem Kondensat enthält, und der Massenprozentsatz der Verbindung (A) bezogen auf die Gesamtmasse der Verbindungen (A) und (C), der durch [Verbindung (A)]/[Verbindungen (A) und (C)] x 100 dargestellt ist, in der Zusammensetzung (mit der Maßgabe, dass in einem Fall, bei dem die Zusammensetzung das teilweise hydrolysierte Cokondensat enthält, bezogen auf das teilweise hydrolysierte Cokondensat die Mengen der Verbindungen (A) und (C) vor der hydrolytischen Cokondensationsreaktion für die Berechnung des Massenprozentsatzes verwendet werden) von 5 bis 70 Massen-% beträgt.

7. Substrat mit einem wasserabweisenden Film nach einem der Ansprüche 3 bis 6, bei dem das Peakverhältnis von [-CF₂O-]/[-CF₂-], das durch ein Röntgenphotoelektronenspektrometer an der Oberfläche des wasserabweisenden Films gemessen worden ist, von 0,1 bis 10,0 beträgt.

8. Substrat mit einem wasserabweisenden Film nach einem der Ansprüche 3 bis 7, bei dem das Peakverhältnis von F1s/Si2p, das durch eine Röntgenphotoelektronenspektroskopie (ESCA) an der Oberfläche des wasserabweisenden Films gemessen worden ist, von 1,5 bis 7,0 beträgt, und Ra (Oberflächenrauheit), die durch ein Rastersondenmikroskop (SPM) gemessen worden ist, von 0,1 bis 5,0 nm beträgt.

9. Verfahren zur Herstellung des Substrats mit einem wasserabweisenden Film nach einem der Ansprüche 3 bis 8, das einen Schritt des Aufbringens der Zusammensetzung zur Bildung eines wasserabweisenden Films nach Anspruch 1 oder 2 auf die Oberfläche eines Substrats oder auf die Oberfläche einer Schicht zur Bildung einer unteren Schicht der äußersten Schicht in dem wasserabweisenden Film, die im Vorhinein auf der Oberfläche eines Substrats gebildet worden ist, und dann Aushärten zur Bildung einer wasserabweisenden Schicht umfasst.

10. Verfahren zur Herstellung des Substrats mit einem wasserabweisenden Film nach einem der Ansprüche 4 bis 8, das einen Schritt des Aufbringens einer Zusammensetzung zur Bildung einer Zwischenschicht auf die Oberfläche eines Substrats und dann Aushärten zur Bildung einer Zwischenschicht, die vorwiegend aus Siliziumoxid hergestellt ist, und einen Schritt des Aufbringens der Zusammensetzung zur Bildung eines wasserabweisenden Films nach Anspruch 1 oder 2 auf die Oberfläche der Zwischenschicht und dann Aushärten zur Bildung einer wasserabweisenden Schicht umfasst.

11. Gegenstand für ein Transportgerät oder -fahrzeug, der das Substrat mit einem wasserabweisenden Film nach einem der Ansprüche 3 bis 7 umfasst.

## Revendications

1. Composition pour former un film hydrofuge, la composition comprenant
(I) un composé (A) et un composé (B), ou
(II) un co-condensat partiellement hydrolysé de :
(i) un composé représenté par la formule (1a) et/ou un condensat partiellement hydrolysé de celui-ci ; et
(ii) un composé représenté par la formule (2a) et/ou un condensat partiellement hydrolysé de celui-ci,
dans laquelle le composé (A) est au moins un composé organique du silicium fluoré ne contenant pas d'atome d'oxygène d'éther et choisi dans l'ensemble constitué par un composé représenté par la formule (1a) suivante, un condensat partiellement hydrolysé de celui-ci, et un composé représenté par la formule (1b) suivante,
R^{f1}-y-Si(R¹¹)ᵣ(X¹)₃₋ᵣ (1a) 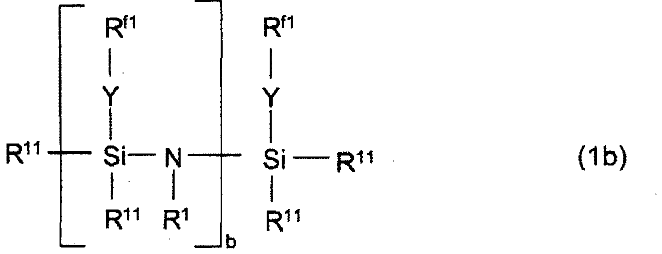
(où, dans les formules (1a) et (1b), R^{f1} est un groupe perfluoroalkyle en C₁ à C₇ de formule CF₃(CF₂)₁- dans laquelle 1 est un entier de 0 à 6, qui ne contient pas d'atome d'oxygène d'éther entre des atomes de carbone-carbone, Y est un groupe organique divalent en C₁ à C₆ qui ne contient pas d'atome de fluor, chaque R¹¹ est indépendamment un atome d'hydrogène ou un groupe hydrocarboné en C₁ à C₆ ne contenant pas d'atome de fluor, chaque X¹ est indépendamment un atome d'halogène, un groupe alcoxy ou un groupe isocyanate, r est un entier de 0 à 2, R¹ est un atome d'hydrogène ou un groupe hydrocarboné en C₁ à C₃ ne contenant pas d'atome de fluor, et b est un entier de 1 à 100),
et dans laquelle le composé (B) est au moins un composé organique du silicium fluoré contenant un atome d'oxygène d'éther, choisi dans l'ensemble constitué par un composé représenté par la formule (2a) suivante, un condensat partiellement hydrolysé de celui-ci, et un composé représenté par la formule (2b) suivante,
R^{f2}-W-Z-Si (R¹²)ₚ(X²)₃₋ₚ (2a) 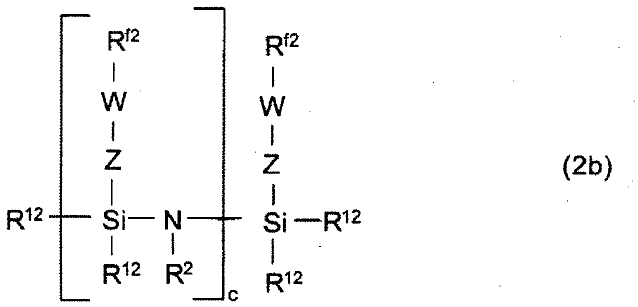
(où, dans les formules (2a) et (2b), R^{f2} est un groupe perfluoroalkyle en C₁ à C₇ de formule CF₃(CF₂)ₗ- où 1 est un entier de 0 à 6, pouvant avoir un atome d'oxygène d'éther inséré entre des atomes de carbone-carbone, W est -O-(CF₂CF₂O)ₐ-CF₂- (où a est un entier de 1 à 200), Z est un groupe organique divalent choisi parmi -CONHC₃H₆- et -CH₂OCO-NHC₃H₆-, chaque R¹² est indépendamment un atome d'hydrogène ou un groupe hydrocarboné en C₁ à C₆ ne contenant pas d'atome de fluor, chaque X² est indépendamment un atome d'halogène, un groupe alcoxy ou un groupe isocyanate, p est un entier de 0 à 2, R² est un atome d'hydrogène ou un groupe hydrocarboné en C₁ à C₃ ne contenant pas d'atome de fluor, et c est un entier de 1 à 100).

2. Composition pour former un film hydrofuge selon la revendication 1, dans laquelle le pourcentage en masse du composé (B) par rapport à la masse totale des composés (A) et (B), représenté par [composé (B)]/[composés (A) et (B)] x 100, dans la composition (sous réserve que, dans le cas où la composition contient un co-condensat partiellement hydrolysé, en ce qui concerne le co-condensat partiellement hydrolysé, les quantités des composés (A) et (B) avant la réaction de co-condensation hydrolytique soient utilisées pour le calcul du pourcentage en masse), est de 10 à 90 % en masse.

3. Substrat avec un film hydrofuge comprenant un substrat et un film hydrofuge formé sur au moins une partie de la surface du substrat, dans lequel le film hydrofuge est composé d'au moins une couche et a, en tant que couche la plus extérieure, une couche hydrofuge formée par utilisation de la composition pour former un film hydrofuge telle que définie dans la revendication 1 ou 2.

4. Substrat avec un film hydrofuge selon la revendication 3, dans lequel le film hydrofuge a en outre une couche intermédiaire constituée principalement de silice entre le substrat et la couche hydrofuge.

5. Substrat avec un film hydrofuge selon la revendication 4, dans lequel la couche intermédiaire est une couche formée par utilisation d'une composition pour former une couche intermédiaire qui contient au moins un composé (C) choisi dans l'ensemble constitué par un composé représenté par la formule (3) suivante, son condensat partiellement hydrolysé, et un perhydropolysilazane,
Si(X³)₄ (3)
(dans la formule (3), chaque X³ est indépendamment un atome d'halogène, un groupe alcoxy ou un groupe isocyanate).

6. Substrat avec un film hydrofuge selon la revendication 5, dans lequel la composition pour former une couche intermédiaire contient en outre le composé (A) ou contient, à la place du composé (C), un co-condensat partiellement hydrolysé du composé représenté par la formule (3) et/ou son condensat partiellement hydrolysé, et le composé représenté par la formule (1a) et/ou son condensat partiellement hydrolysé, et le pourcentage en masse du composé (A) par rapport à la masse totale des composés (A) et (C), représenté par [composé (A)]/[composés (A) et (C)] x 100, dans la composition (sous réserve que, dans le cas où la composition contient le co-condensat partiellement hydrolysé, en ce qui concerne le co-condensat partiellement hydrolysé, les quantités des composés (A) et (C) avant la réaction de co-condensation hydrolytique soient utilisées pour le calcul du pourcentage en masse), est de 5 à 70 % en masse.

7. Substrat avec un film hydrofuge selon l'une quelconque des revendications 3 à 6, dans lequel le rapport de pics de [-CF₂O-]/[-CF₂-], tel que mesuré par un spectromètre photoélectronique à rayons X à la surface du film hydrofuge, est de 0,1 à 10,0.

8. Substrat avec un film hydrofuge selon l'une quelconque des revendications 3 à 7, dans lequel le rapport de pics de Fls/Si2p, tel que mesuré par spectroscopie photoélectronique aux rayons X (ESCA) à la surface du film hydrofuge, est de 1,5 à 7,0, et la rugosité de surface Ra, telle que mesurée par un microscope en champ proche (SPM), est de 0,1 à 5,0 nm.

9. Procédé pour produire le substrat avec un film hydrofuge tel que défini dans l'une quelconque des revendications 3 à 8, qui comprend une étape d'application de la composition pour former un film hydrofuge telle que définie dans la revendication 1 ou 2 sur la surface d'un substrat ou sur la surface d'une couche pour constituer une couche inférieure de la couche la plus extérieure dans le film hydrofuge, formé au préalable sur la surface d'un substrat, suivie d'un durcissement pour former une couche hydrofuge.

10. Procédé pour produire le substrat avec un film hydrofuge tel que défini dans l'une quelconque des revendications 4 à 8, qui comprend une étape d'application d'une composition pour former une couche intermédiaire sur la surface d'un substrat, suivie d'un durcissement pour former une couche intermédiaire constituée principalement de silice, et une étape d'application de la composition pour former un film hydrofuge telle que définie dans la revendication 1 ou 2 sur la surface de la couche intermédiaire, suivie d'un durcissement pour former une couche hydrofuge.

11. Article pour équipement de transport, qui comprend le substrat avec un film hydrofuge tel que défini dans l'une quelconque des revendications 3 à 7.
